(19) Europäisches Patentamt
European Patent Office
Office européen des brevets

(11) EP 3 915 983 A1

(12) EUROPEAN PATENT APPLICATION

(43) Date of publication:
**01.12.2021 Bulletin 2021/48**

(21) Application number: **20305545.4**

(22) Date of filing: **26.05.2020**

(51) Int Cl.:
*C07D 417/08* (2006.01) *C07D 413/08* (2006.01)
*C07D 417/12* (2006.01) *A61K 31/427* (2006.01)
*A61K 31/422* (2006.01) *A61K 31/4439* (2006.01)
*A61P 39/00* (2006.01) *A61P 39/02* (2006.01)
*A61P 25/28* (2006.01) *A61P 35/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Centre National de la Recherche Scientifique 75016 Paris (FR)**
- **Université de Strasbourg 67000 Strasbourg (FR)**
- **Etat Français représenté par la Direction Centrale Du Service de Santé des Armées 75015 Paris (FR)**

(72) Inventors:
- **BAATI, Rachid 67087 Strasbourg Cedex 2 (FR)**
- **DIAS, José 91223 Brétigny sur Orge (FR)**
- **NACHON, Florian 91220 Brétigny sur Orge (FR)**
- **NIMMAKAYALA, Mallikarjuna Reddy 67087 Strasbourg Cedex 2 (FR)**

(74) Representative: **Lavoix 2, place d'Estienne d'Orves 75441 Paris Cedex 09 (FR)**

# (54) PYRIDINE-THIAZOLE-OXIME AND PYRIDINE-OXAZOLE-OXIME DERIVATIVES AS REACTIVATORS OF ORGANOPHOSPHOROUS NERVE AGENT (OPNA)-INHIBITED HUMAN ACETYLCHOLINESTERASE FOR THE TREATMENT OF NERVOUS AND/OR RESPIRATORY FAILURE AFTER INTOXICATION WITH OPNA

(57) The present invention relates to a compound of formula (I):

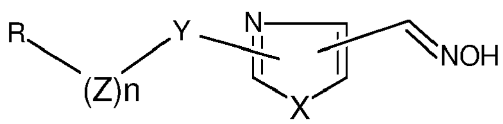

(I)

wherein X is O or S; Y is $-CH_2-CH_2-$, -C=C- or -CH=CH-; Z is $-CH_2-$; n is an integer from 0 to 4; and R is an alkyl group, an aryl, a heteroaryl, a cycloalkyl, a heterocyclyl, a biomolecule, a fluorescent probe, or a group -N(R1 )(R2); wherein R1 and R2 are each independently H, an alkyl group, an aryl, a heteroaryl or a cycloalkyl.

It also relates to a pharmaceutical composition comprising at least one compound of formula (I) and at least one pharmaceutically acceptable support.

Finally, it relates to the use of such a compound as a medicine, preferably in the treatment of a nervous and/or respiratory failure due to intoxication with at least one organo-phosphorous nerve agent (OPNA); in the treatment of neurological diseases such as Alzheimer's disease; and/or in the treatment of cancer.

The compounds act as reactivators of OPNA-inhibited hAChE (human acetylcholinesterase).

The present description discloses the syntheses of exemplary compounds as well as experimental data on the in vitro reactivation of human acetylcholinesterase (hAChE) by the compounds of the invention (e.g. pages 28 to 56; examples 1 and 2; compounds; tables 1 to 3).

Exemplary compounds are e.g.:

EP 3 915 983 A1

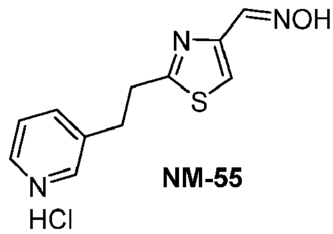

NM-55

or

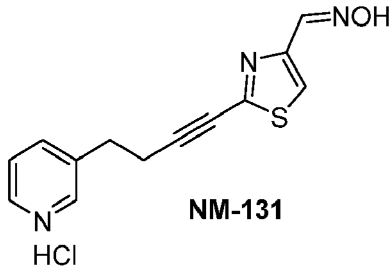

NM-131

## Description

The present invention relates to novel compounds having a thiazoloxime or oxazoloxime scaffold. Such compounds may be useful for many therapeutic and non-therapeutic applications. The invention also relates to compositions, notably pharmaceutical compositions, comprising said compounds, and their use.

Organophosphorous nerve agents (OPNA) are extremely toxic compounds that comprise chemical warfare agents (CWA) including sarin, soman, cyclosarin, tabun, methylphosphonothioate (VX) and pesticides such as paraoxon, parathion and tetraethyl pyrophosphate (TEPP). Their acute toxicity results from the irreversible inhibition of acetylcholinesterase (AChE) through phosphylation of its catalytic serine, which results in the inability of the enzyme to hydrolyze acetylcholine (ACh). Accumulation of this neurotransmitter at cholinergic synapses occurs, leading to a permanent saturation of the muscarinic and nicotinic receptors which ultimately results in seizure and respiratory arrest. Depending on the class of OPNA and on the administrated dose, death can occur within a few minutes.

Due to the similarity between the chemical precursors of CWA and pesticides, and to the relatively simple chemistry involved in their synthesis, efforts to control the proliferation of these agents have proved of limited success. Therefore, the development of effective measures to counteract OPNA poisoning remains a challenging issue to protect and treat both civilian and military populations. The current treatment for OPNA poisoning consists in the administration of a combination of atropine (antimuscarinic agent) and diazepam (anticonvulsant drug), to limit convulsions, and of a standard pyridinium oxime (pralidoxime, trimedoxime, HI-6, obidoxime, or HLö-7) to reactivate AChE. Oximes exert their action on OPNA-inhibited AChE by attacking the phosphorous atom of the phosphylated serine, leading to the removal of the phosphonate and restoration of the enzyme's catalytic activity. However, it has been demonstrated that the current therapy results in unequal efficiency, and none of these oximes offer broad efficacy across the different OPNAs. Further limitations of oxime-based therapy include inability to cross the blood-brain barrier (BBB), inability to reactivate the "aged" enzyme, and rapid clearance from the circulation when tested *in vivo.* Animal model studies and recent clinical trials using pesticide poisoned individuals have shown uneven clinical benefits of these oximes, and even harm, so their true efficacy as antidotes has been debated at the World Health Organisation.

To overcome the disadvantages of the current medication, the development of new broad spectrum and bioavailable centrally active drugs is of crucial importance.

Over the past decades, there has been a growing interest in the development of non-ionic oximes reactivators of OPNA-inhibited hAChE (human AChE) to increase BBB permeability. For example, uncharged hybrid reactivators bearing 3-hydroxy-2-pyridinaldoxime as nucleophilic moiety and a peripheral site AChE ligand, exhibited increased affinity for the phosphylated enzyme, a large spectrum of reactivation and the ability to cross efficiently the BBB *in vitro.* Beside these discoveries, others heterocyclic, aromatic and acyclic nucleophilic oximes, as well as uncharged acetamido bis-oximes have been developed as antidotes, with more or less success in the reactivation of OPNA-inhibited AChE. Recently, unusual non-oxime non-ionic new functional groups such as Mannich phenols that are capable of reactivating OPNA-inhibited AChE have been reported by Katz, Cadieux and De Koning (Katz et al, ChemBioChem. 2015, 16, 2205-2215; de Koning et al, Eur. J. Med. Chem. 2018, 157,151-160; Cadieux et al, Chemico-Biological Interactions 2016, 259, 133-141). However, the mechanism of the reactivation is still unclear, and the development of these molecules is hampered by their low stability in biological media. Recent findings have demonstrated the ability of a zwitterionic, centrally acting, brain penetrating oxime to reverse severe symptoms and rapidly reactivate sarin- and paraoxon inhibited AChE *in vivo.*

It is further obvious that the above-mentioned compounds are accessed only after tedious, non-flexible and lengthy multistep chemical synthesis due to their increased structural complexity.

Despite these innovative strategies for the development of reactivators, efforts towards shorter and more convergent synthetic routes to innovative broad spectrum and centrally effective antidotes are still needed. There is thus a remaining need for chemical compounds efficient in therapeutic applications, particularly against OPNA intoxications, with a broad spectrum and centrally effective. These compounds have to be quick and easy to synthetize.

Surprisingly, the inventors have now discovered that specific compounds, having a thiazoloxime or an oxazoloxime scaffold, fulfill these needs.

Indeed, such compounds are quick and very easy to produce thanks to a late-stage Sonogashira cross-coupling reaction of bromothiazoloximes isomers, which leads to a short and expedient synthesis, without using protecting groups for the sensitive oximes. The compounds present very interesting properties: they have a low molecular weight, and exhibit a quite simple molecular structural design and a broad spectrum of reactivation of OPNA-inhibited AChE, especially with increased efficacy for VX and paraoxon, and a good potency against sarin.

Notably, these compounds may be used as antidotes against OPNA intoxications or as detoxifying or decontamination agents against organophosphorus compounds, thanks to their effective and fast reactivation of hAChE without denaturing the same. They may also be used in the treatment of neurodegenerative diseases such as Alzheimer's disease. Finally, particularly the oxime compounds of the invention may be used as histone deacetylases (HDAC) inhibitors; consequently, they may be used in the treatment of cancer.

Thus, a first object of the present invention is a compound of formula (I):

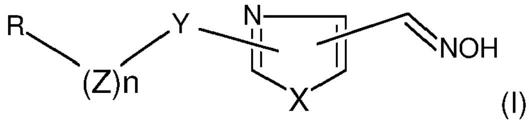

(I)

wherein the different groups are as defined in the detailed description below.

Another object of the present invention is a process for preparing the compounds of formula (I), especially by a Sonogashira reaction, as detailed below.

Another object of the present invention is a pharmaceutical composition comprising at least one compound of formula (I) and at least one pharmaceutically acceptable support.

Another object of the invention is a compound according to the invention, for use as a medicine.

A further object of the invention is a compound according to the invention for use in the treatment of a nervous and/or respiratory failure due to intoxication with at least one organophosphorous nerve agent.

Still a further object of this invention is a compound according to the invention for use in the treatment of neurological diseases such as Alzheimer's disease.

Still a further object of this invention is a compound according to the invention for use in the treatment of cancer.

A first object of the present invention is a compound of formula (I), or one of its pharmaceutically acceptable salts:

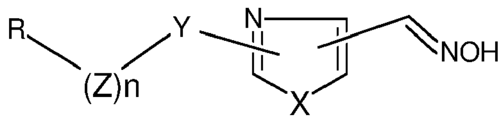

(I)

wherein:

X is O or S;
Y is $-CH_2-CH_2-$, $-C{\equiv}C-$ or $-CH=CH-$;
Z is $-CH_2-$,
n is an integer from 0 to 4; and
R is an alkyl group, an aryl, a heteroaryl, a cycloalkyl, a heterocyclyl, a biomolecule, a fluorescent probe, or a group -N(R1)(R2), wherein R1 and R2 are each independently H, an alkyl group, an aryl, a heteroaryl or a cycloalkyl.

When X is O, then formula (I) is an oxazoloxime scaffold.
When X is S, then formula (I) is a thiazoloxime scaffold.

By "pharmaceutically acceptable salt", it is meant any salt of a compound of formula (I) with an acid or a base. Preferably, the pharmaceutically acceptable salt is a chlorhydrate salt. Such a salt may be obtained by using HCl. More preferably, the heteroaryl group of R is a nitrogen atom, which is complexed with HCl.

Preferably, the compound of the invention is a salt of a compound of formula (I), more preferably a chlorhydrate salt of a compound of formula (I).

The compound of formula (I) may be labeled with one or more isotopes such as $^{15}N$, $^{18}O$, $^{2}H$ or $^{3}H$. Preferably the compound is labeled on the =N-OH group, with $^{15}N$. Indeed, such a stable, non-toxic and non-radioactive isotope would allow *in vivo* and *in vitro* biological studies.

By "alkyl", it is meant a linear hydrocarbon group preferably comprising from 1 to 20 carbon atoms, in particular from 1 to 15 carbon atoms, or a branched or cyclic hydrocarbon group comprising from 3 to 20 carbon atoms. Examples of alkyl groups include methyl, ethyl, n-propyl, isopropyl, n-butyl, isobutyl, tert-butyl, n-pentyl, isopentyl, n-hexyl, n-tridecyl, cyclohexyl and cyclohexylmethyl groups, and preferably ethyl, propyl, n-hexyl, n-tridecyl, cyclohexyl or cyclohexylmethyl group.

By "aryl", it is meant a monocyclic or polycyclic aromatic hydrocarbon group, which may be optionally substituted. Preferably, the aryl group is a phenyl, or a polycyclic aromatic hydrocarbon (PAH). A preferred PAH is pyrene. The aryl is preferably not substituted.

By "heteroaryl", it is meant an aryl group in which at least one carbon atom of the aromatic ring is substituted by a heteroatom, and which may be optionally substituted. The heteroatom may be nitrogen, oxygen, phosphorus or sulfur. Preferably the heteroatom is nitrogen. Examples of heteroaryl groups include pyridine, pyrrole, thiophene, furane, pyrimidine, pyrazine, pyridazine, triazole, tetrazine, triazine, imidazole, quinoline, thiazole, oxazole, tetrazole, oxadiazole,

thiadiazole, and isoxazole groups. Preferably, the heteroaryl group is a pyridine group such as 2-, 3- or 4-pyridino, or a quinoline group such as a 4-quinolinyl. The heteroaryl is preferably not substituted.

A "cycloalkyl" is refers to a monocyclic or polycyclic saturated hydrocarbon group, which may be optionally substituted.

A "heterocyclyl" is refers to a monocyclic or polycyclic saturated hydrocarbon group in which at least one carbon atom of the ring is substituted by a heteroatom, and which may be optionally substituted. The heteroatom may be nitrogen, oxygen, or sulfur. Preferably, the heterocyclic group is morpholino, pyrazolidine, oxathiolane, tetrahydrofuran, dioxolane, piperidine, piperazine, thiomorpholine, tetrahydropyrane, oxetane or azetidine, such as 4-tetrahydropyrano or 3-oxetano or 3-azetidino. The heterocyclyl may be substituted or not.

By "biomolecule", it is meant a sugar moiety, a peptide moiety, an antibody, a virus, a DNA, a RNA or a protein moiety. The sugar moiety may be for example a glucose, fructose or sucrose moiety. A peptide moiety is a moiety typically comprising 1 to 50 amino acids. A protein moiety is a moiety typically comprising at least 51 amino acids, preferably from 60 to 500 amino acids.

By "fluorescent probe" it is meant a chemical function or a fluorophore endowed with fluorescent properties. The fluorescent moiety may be for example a fluoresceine, boron dipyrromethene (BODIPY), a coumarine, a cyanine, an Alexa Fluor, an acridine, a fluorone, a squaraine, a phenanthridine, a cyanine, an oxazine, a perylene, an anthracene or rhodamine moiety.

Preferably, R is a heteroaryl or a group -N(R1)(R2), wherein R1 and R2 are each independently H or a heteroaryl. Preferably, the heteroaryl group is a pyridine group such as 2-, 3- or 4-pyridino, or a quinoline group such as a 4-quinolinyl. Preferably, R is a pyridine group such as 2-, 3- or 4-pyridino.
Preferably, according to another embodiment, R is a group -N(R1)(R2), wherein R1 is H, and R2 is a heteroaryl, preferably a quinoline group such as a 4-quinolinyl.

Preferably, the -Y-(Z)n-R group is in position 2.
According to a first embodiment, preferably, the oxime group is in 4 position, and the compound of the invention is a 2-substituted-4-oxime-(oxazole or thiazole) of formula (II), or one of its pharmaceutically acceptable salts:

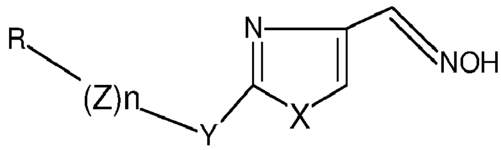

(II)

According to a second embodiment, preferably, the oxime group is in 5 position, and the compound of the invention is a 2-substituted-5-oxime-(oxazole or thiazole) of formula (III), or one of its pharmaceutically acceptable salts:

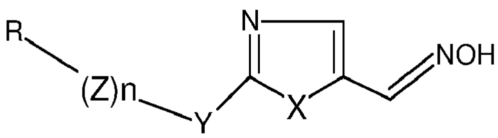

(III)

According to said first embodiment, it is preferred that the compound of formula (I) is a 2-substituted-4-oxime-(oxazole or thiazole) of formula (II), or one of its pharmaceutically acceptable salts:

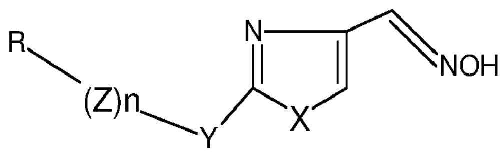

(II)

Preferably, X is S. In such a case, the compounds of formula (I) are 2-substituted-4-oxime-1,3-thiazoles.

X may also be O. In such a case, the compounds of formula (I) are 2-substituted-4-oxime-1,3-oxazoles.

Preferably, Y is $-CH_2-CH_2-$ or -C≡C-, and n is 0, 1 or 2, preferably n is 0 or 2.
Preferably, R is a heteroaryl or a group -N(R1)(R2). Preferably said heteroaryl is not substituted. Preferably, said heteroaryl is a pyridine group such as 2-, 3- or 4-pyridino. Preferably, said group -N(R1)(R2) is such that R1 is H, and R2 is a heteroaryl, preferably not substituted, preferably a quinoline group such as a 4-quinolinyl.

Preferably, according to said first embodiment, the compounds of formula (II) are such that:

- X is S;
- Y is $-CH_2-CH_2-$ or -C≡C-,
- n is 0, 1 or 2, preferably n is 0 or 2; and
- R is a heteroaryl or a group -N(R1)(R2), preferably said heteroaryl is not substituted and is a pyridine group such as 2-, 3- or 4-pyridino; or R1 is H and R2 is a heteroaryl, preferably not substituted, preferably a quinoline group such as a 4-quinolinyl.

Preferably, according to said first embodiment, the compounds of formula (II) are also such that:

- X is O;
- Y is $-CH_2-CH_2-$ or -C≡C-,
- n is 0, 1 or 2, preferably n is 0 or 2; and
- R is a heteroaryl, preferably said heteroaryl is not substituted and is a pyridine group such as 2-, 3- or 4-pyridino.

Preferably, when Y is $-CH_2-CH_2-$, n is preferably 0 or 2, and R is a heteroaryl, preferably not substituted, and is a pyridine group such as 2-, 3- or 4-pyridino.

Preferably, the compound of formula (II) or one of its pharmaceutically acceptable salts is chosen from the following compounds:

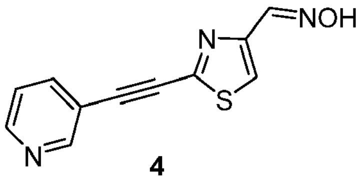

**4**

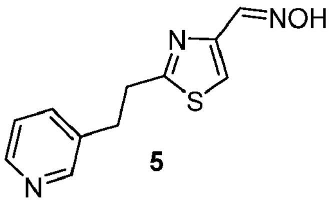

**5**

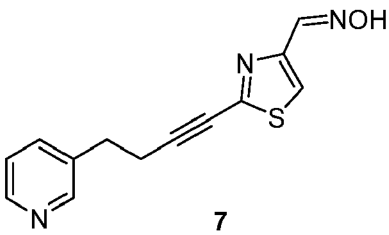

**7**

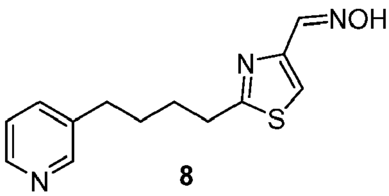

**8**

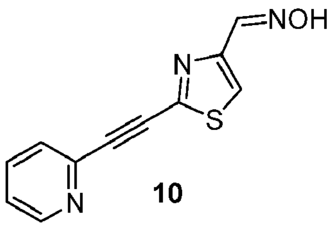

10

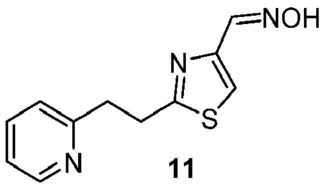

11

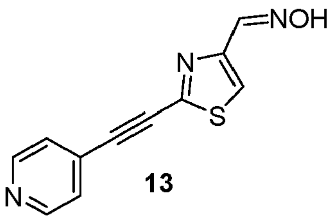

13

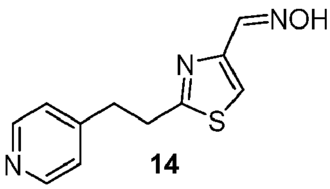

14

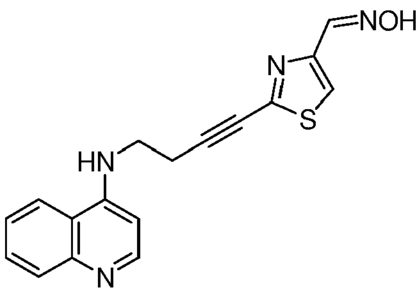

23

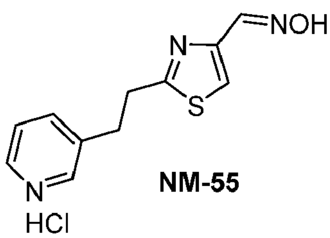

NM-55

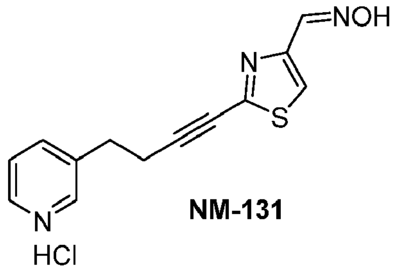

NM-131

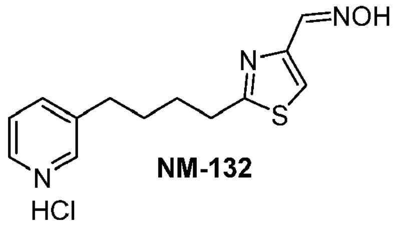

NM-132

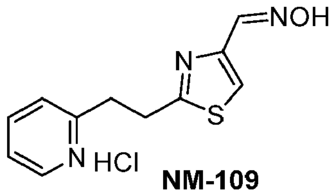

NM-109

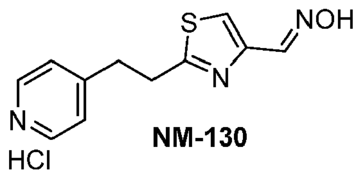

NM-130

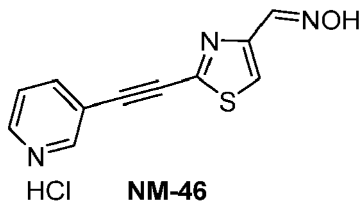

NM-46

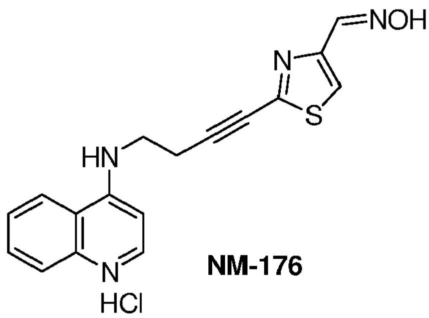

NM-176

and

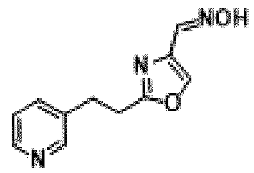

According to said second embodiment, it is preferred that the compound of formula (I) is a 2-substituted-5-oxime-(oxazole or thiazole) of formula (III):

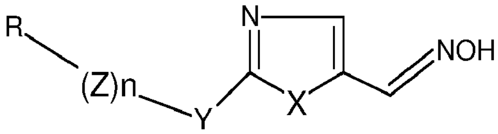

(III)

Preferably, X is S. In such a case, the compounds of formula (I) are 2-substituted-5-oxime-1,3-thiazoles.

X may also be O. In such a case, the compounds of formula (I) are 2-substituted-5-oxime-1,3-oxazoles.

Preferably, Y is $-CH_2-CH_2-$ or -C≡C-, and n is 0, 1 or 2, preferably n is 0 or 2.
Preferably, R is a heteroaryl or a group -N(R1)(R2), wherein R1 and R2 are each independently H or a heteroaryl. Preferably said heteroaryl is not substituted. Preferably, said heteroaryl is a pyridine group such as 2-, 3- or 4-pyridino, or a quinoline group such as a 4-quinolinyl.
Preferably, R is a pyridine group such as 2-, 3- or 4-pyridino.

Preferably, according to another embodiment, R1 is H, and R2 is a heteroaryl, preferably a quinoline group such as a 4-quinolinyl.

Preferably, according to said second embodiment, the compounds of formula (III) are such that:

- X is S;
- Y is $-CH_2-CH_2-$ or -C≡C-,
- n is 0, 1 or 2, preferably n is 0 or 2; and
- R is a heteroaryl or a group -N(R1)(R2), preferably said heteroaryl is not substituted and is a pyridine group such as 2-, 3- or 4-pyridino; or R1 is H and R2 is a heteroaryl, preferably not substituted, preferably a quinoline group such as a 4-quinolinyl.

Preferably, according to said second embodiment, the compounds of formula (III) are also such that:

- X is O;
- Y is $-CH_2-CH_2-$ or -C≡C-,
- n is 0, 1 or 2, preferably n is 0 or 2; and
- R is a heteroaryl, preferably said heteroaryl is not substituted and is a pyridine group such as 2-, 3- or 4-pyridino.

Preferably, when Y is $-CH_2-CH_2-$, n is preferably 0 or 2, and R is a heteroaryl, preferably not substituted, and is a pyridine group such as 2-, 3- or 4-pyridino.

Preferably, the compound of formula (III) is chosen from the following compounds:

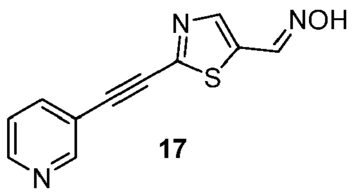

**17**

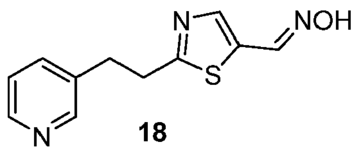

**18**

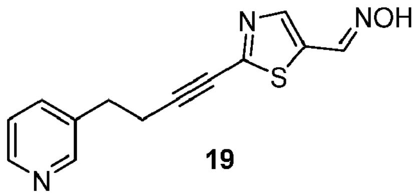

19

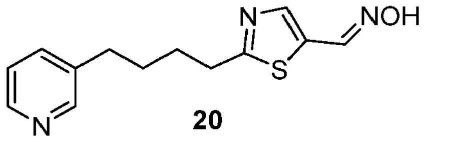

**20**

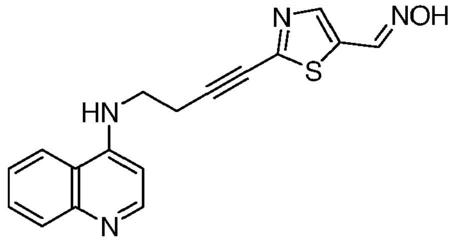

**22**

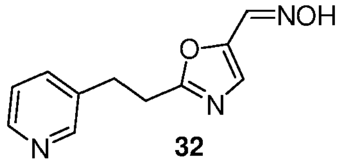

**32**

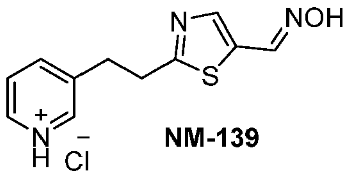

**NM-139**

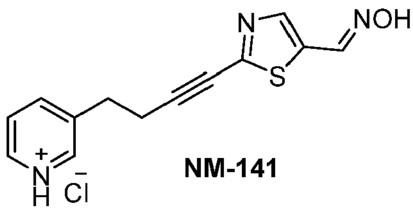

**NM-141**

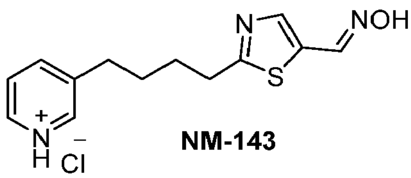

**NM-143**

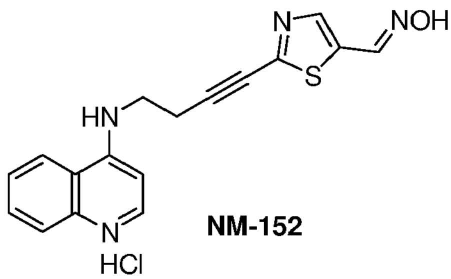

**NM-152**

and

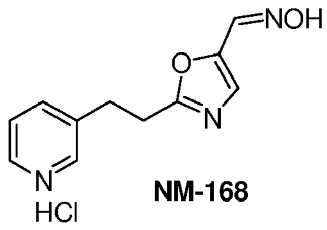

NM-168

Preferably, the oxime group is in position 2.
According to a third embodiment, preferably, the -Y-(Z)n-R group is in 5 position, and the compound of formula (I) is a 5-substituted-2-oxime-(oxazole or thiazole) of formula (IV):

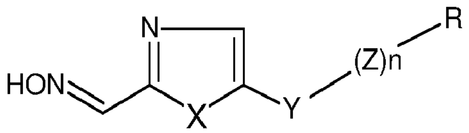

(IV)

Preferably, X is S.
In such a case, the compounds of formula (I) are 5-substituted-2-oxime-1,3-thiazoles.
Preferably, X is O.
In such a case, the compounds of formula (I) are 5-substituted-2-oxime-1,3-oxazoles.
Preferably, Y is $-CH_2-CH_2-$ or -C≡C-, and n is 0, 1 or 2, preferably n is 0 or 2.
Preferably, R is a heteroaryl. Preferably said heteroaryl is not substituted. Preferably, said heteroaryl is a pyridine group such as 2-, 3- or 4-pyridino.
Preferably, according to said third embodiment, the compounds of formula (IV) are such that:

- X is S;
- Y is $-CH_2-CH_2-$ or -C≡C-,
- n is 0, 1 or 2, preferably n is 0 or 2; and
- R is a heteroaryl, preferably said heteroaryl is not substituted and is a pyridine group such as 2-, 3- or 4-pyridino.

Preferably, when Y is $-CH_2-CH_2-$, n is preferably 0 or 2, and R is a heteroaryl, preferably not substituted, and is a pyridine group such as 2-, 3- or 4-pyridino.
Preferably, the compound of formula (IV) is chosen from the following compounds:

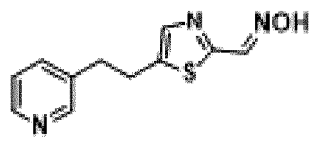

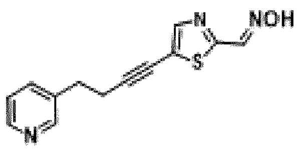

and

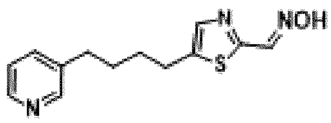

According to a fourth embodiment, preferably, the -Y-(Z)n-R group is in 4 position, and the compound of formula (I) is a 4-substituted-2-oxime-(oxazole or thiazole) of formula (V):

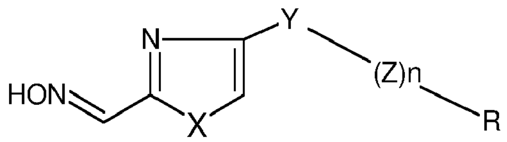
(V)

Preferably, X is S.
In such a case, the compounds of formula (I) are 4-substituted-2-oxime-1,3-thiazoles.
Preferably, X is O.
In such a case, the compounds of formula (I) are 4-substituted-2-oxime-1,3-oxazoles.
Preferably, Y is $-CH_2-CH_2-$ or -C≡C-, and n is 0, 1 or 2, preferably n is 0 or 2.
Preferably, R is a heteroaryl. Preferably said heteroaryl is not substituted. Preferably, said heteroaryl is a pyridine group such as 2-, 3- or 4-pyridino.
Preferably, according to said third embodiment, the compounds of formula (V) are such that:

- X is S;
- Y is $-CH_2-CH_2-$ or -C≡C-,
- n is 0, 1 or 2, preferably n is 0 or 2; and
- R is a heteroaryl, preferably said heteroaryl is not substituted and is a pyridine group such as 2-, 3- or 4-pyridino.

Preferably, when Y is $-CH_2-CH_2-$, n is preferably 0 or 2, and R is a heteroaryl, preferably not substituted, and is a pyridine group such as 2-, 3- or 4-pyridino.
Preferably, the compound of formula (V) is chosen from the following compounds:

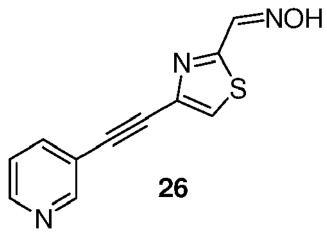
**26**

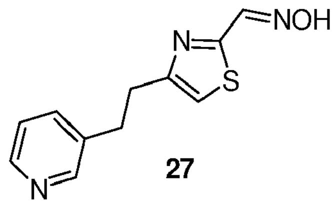
**27**

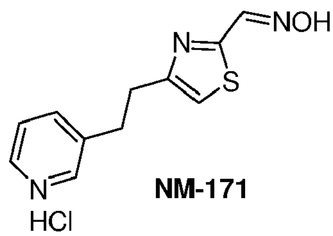
**NM-171**

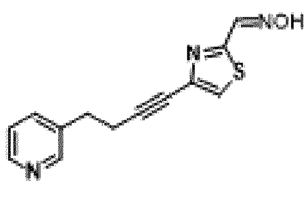

and

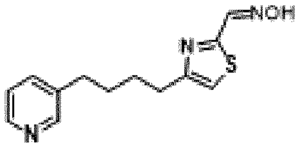

Preferably, the compound of formula (I) is chosen from compounds of formula (II), (III), (IV) and (V).
More preferably, the compound of formula (I) is chosen from:

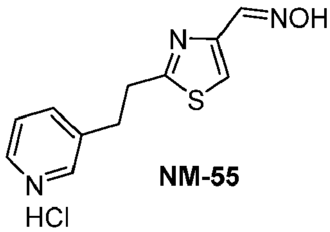

**NM-55**

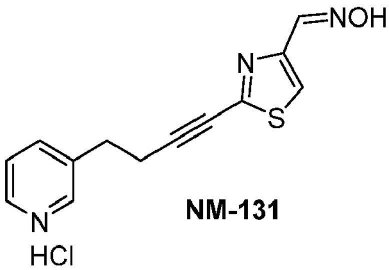

**NM-131**

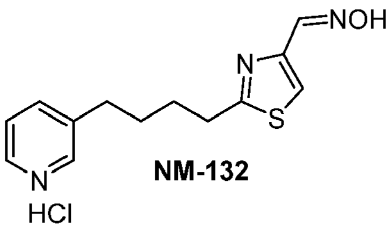

**NM-132**

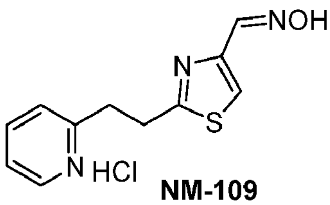

**NM-109**

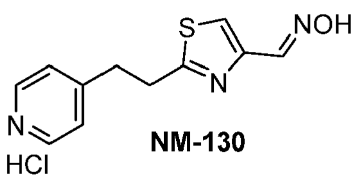

**NM-130**

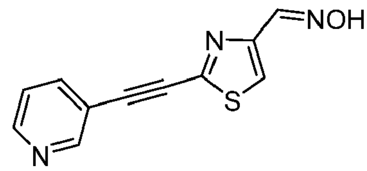

NM-46

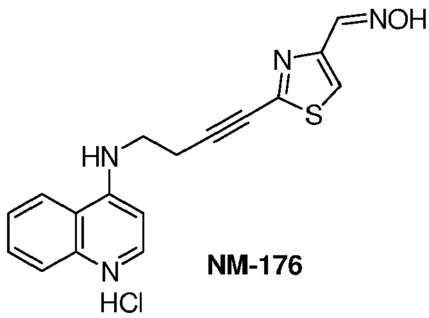

NM-176

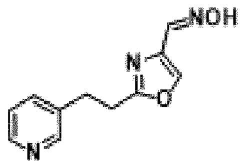

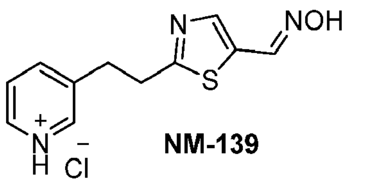

NM-139

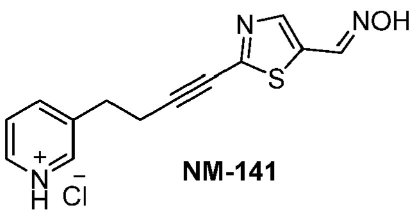

NM-141

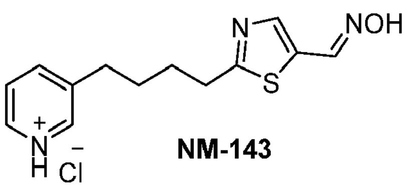

NM-143

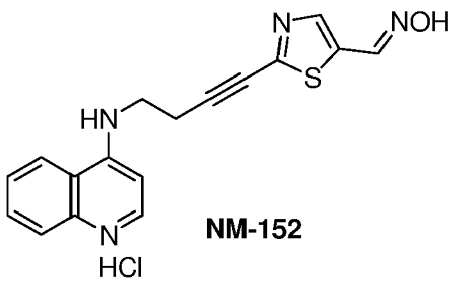

NM-152

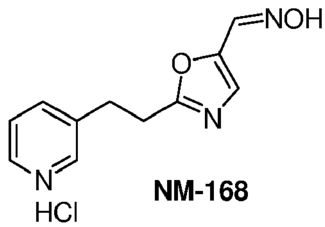

NM-168

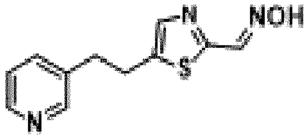

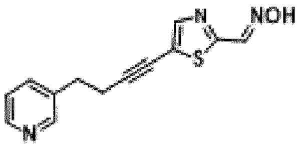

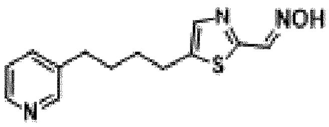

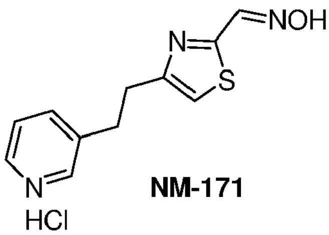

NM-171

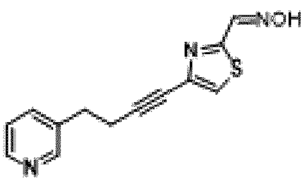

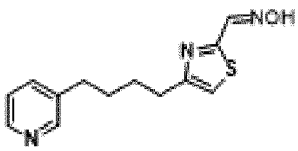

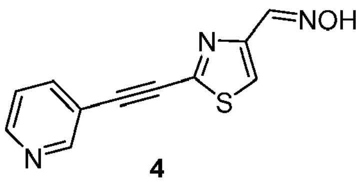

4

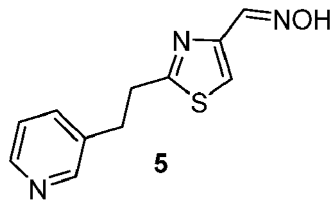

5

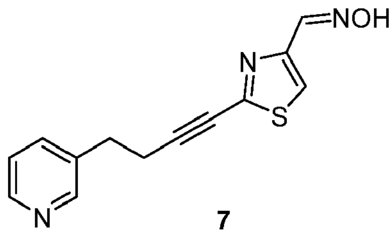

7

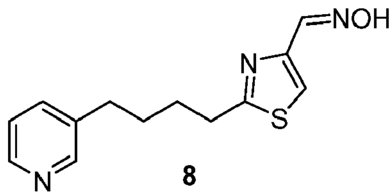

8

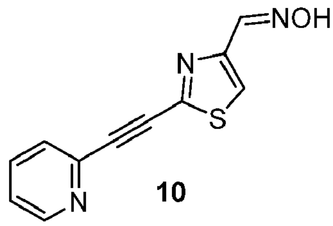

10

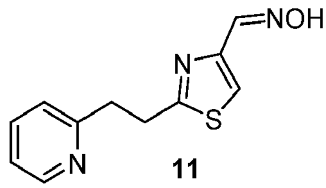

11

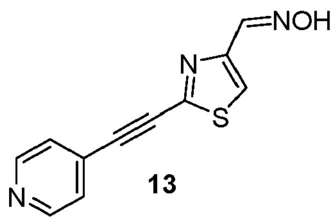

13

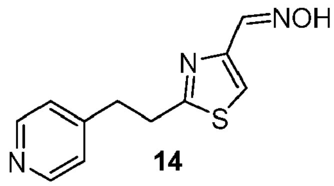

**14**

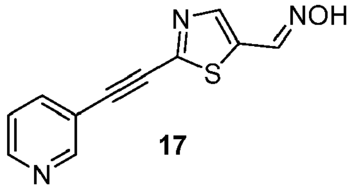

**17**

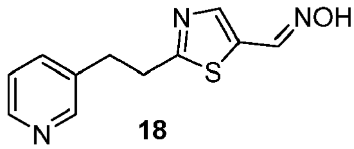

**18**

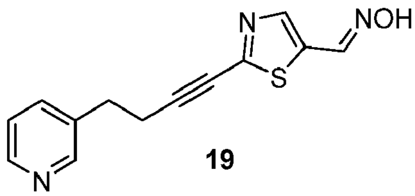

**19**

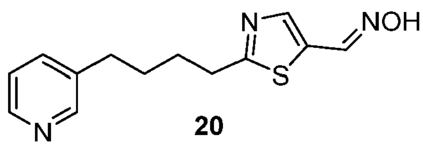

**20**

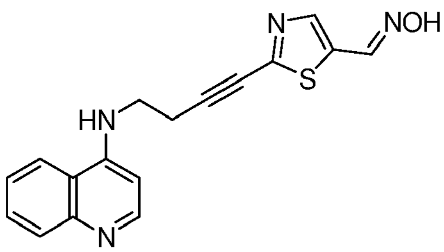

**22**

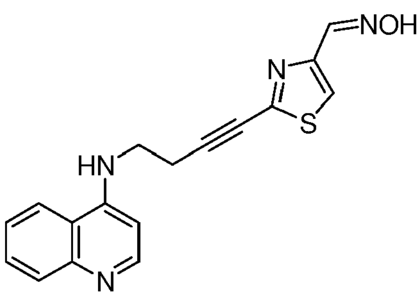

**23**

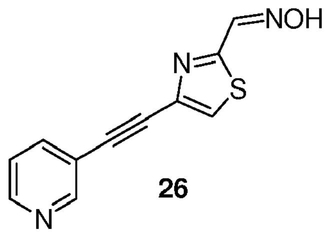

26

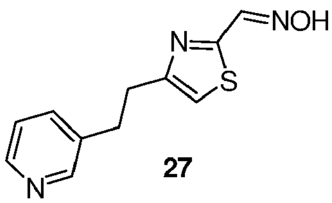

27

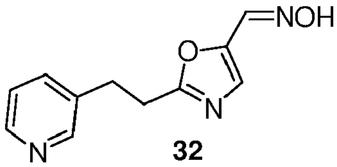

32

More preferably, the compound of formula (I) is chosen from:

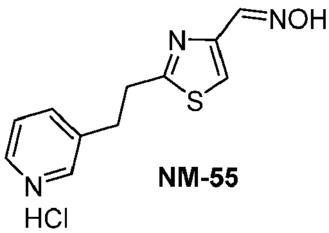

NM-55

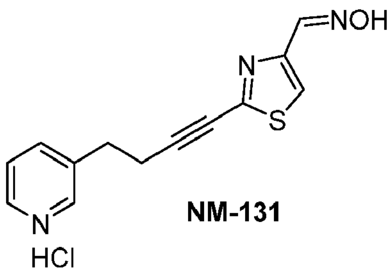

NM-131

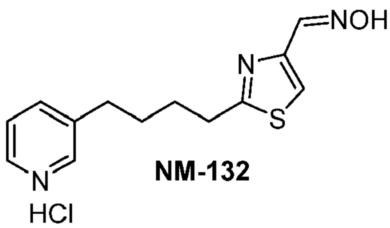

NM-132

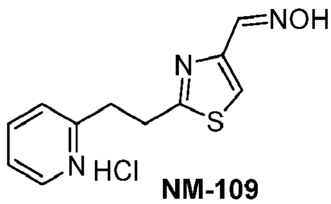

**NM-109**

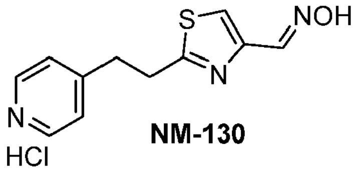

**NM-130**

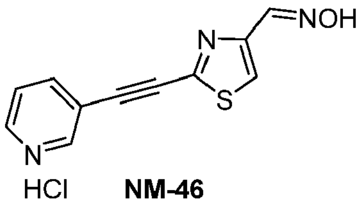

**NM-46**

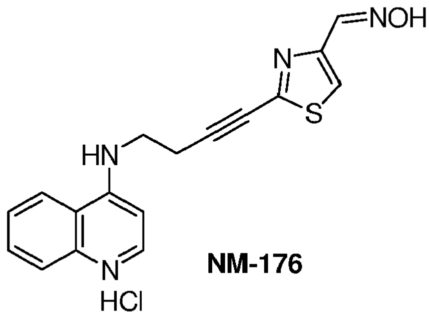

**NM-176**

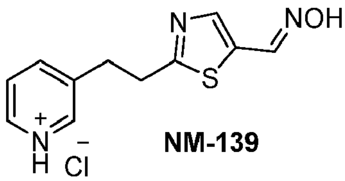

**NM-139**

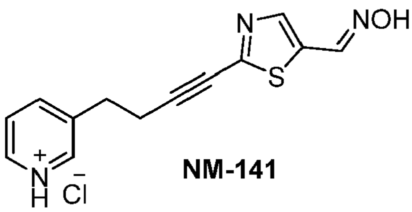

**NM-141**

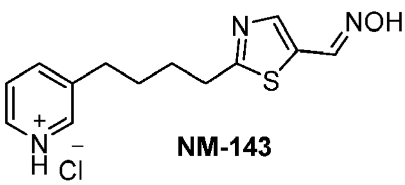

**NM-143**

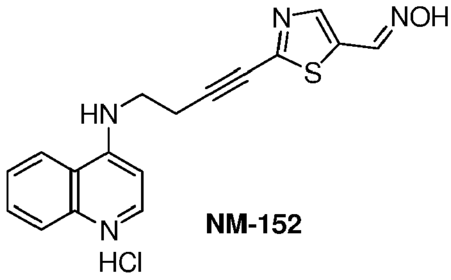

NM-152

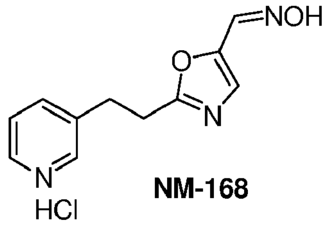

NM-168

and

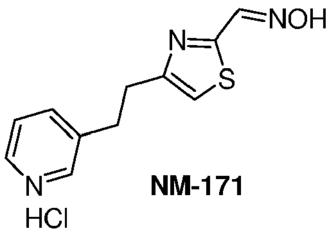

NM-171

Preparation of the compounds of formula (I)

A compound of formula (I) according to the invention may be synthesized by any appropriate method. For example, when n is 0, the compounds of formula (I) may be prepared according to the following scheme :

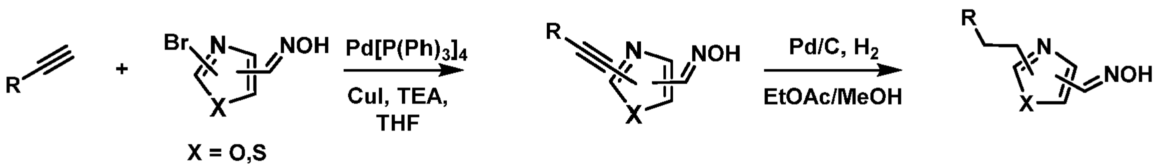


A B C D

Such methods are exemplified in the following examples.

Preferably, the compounds of formula (I) are synthetized as described below. Such a process is chemoselective. Particularly, it does not necessitate any previous protection step of the oxime. Said process comprises a minimal number of steps (one or two), is quickly performed, at ambient temperature.

The main steps are as follows:

The late stage Sonogashira C-C cross-coupling reaction, recently developed by the group of the inventors (Yerri et al. Eur. J. Med .Chem. 2018, 4161-4165) was applied between terminal alkyne **A** and isomers of unprotected bromo-oxime-1,3-thiazole or unprotected isomers of bromo-oxime-1,3-oxazole **B** under palladium catalysis afford the conjugate **C** (which is of formula (I)). Said conjugate is then submitted to hydrogenation with Pd/C catalyst in heterogeneous conditions, to provide the corresponding alkene, and finally the hybrid reactivator **D** (which is of formula (I)).

Compounds C, alkene and D are all according to the invention.

When n is 1, 2, 3 or 4, then the above process is also applicable.

Thus, the present invention also relates to a process for preparing a compound of formula (I), which comprises the following steps:

- a Sonogashira coupling reaction between a terminal alkyne

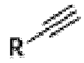

and an isomer of unprotected bromo-oxime-1,3-thiazole or an isomer of unprotected bromo-oxime-1,3-oxazole, preferably under palladium catalysis, to obtain the conjugate

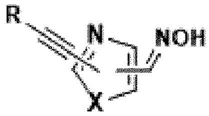

(which is of formula (I));
- optionally, said conjugate is then submitted to hydrogenation, preferably with Pd/C catalyst in heterogeneous conditions, to provide the corresponding alkene, and finally the hybrid reactivator

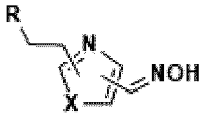

(which is of formula (I)).

Pharmaceutical uses of the compounds of the invention

The compounds of this invention may be used in the treatment of a nervous and/or respiratory failure due to intoxication with at least one organophosphorous nerve agent which may preferably be selected from warfare agents such as O-ethyl S-[2-(diisopropylamino)ethyl] methylphosphonothioate (VX), tabun, sarin, cyclosarin and soman and pesticides such as paraoxon, parathion and tetraethyl pyrophosphate (TEPP). The compounds of the invention may be used in the treatment of a nervous and/or respiratory failure due to intoxication with at least one organophosphorous nerve agent, by virtue of their reactivation potency of organophosphorous inhibited cholinesterases, including acetylcholinesterase and butyrylcholinesterase. These compounds may alternatively be used in the treatment of diseases, which involve a reduced production of acetylcholine that may be overcome by the administration of acetylcholinesterase inhibitors. Examples of such diseases include in particular neurological diseases such as Alzheimer's disease.
These compounds may alternatively be used in the treatment of cancer, thanks to their action as inhibitors of histone deacetylases (HDAC).

The compound of this invention is usually included in a pharmaceutical composition comprising at least one compound according to the invention and a pharmaceutically acceptable support.

The amount of compound of formula (I) in the composition according to the invention may vary in a broad range depending upon the patient, the mode of administration and the expected effect.
The compound or composition according to the invention can be administered orally or non-orally, for instance via topical, parenteral, intramuscular, intravenous, cutaneous, nasal or rectal route.
The pharmaceutical composition of the invention can present different forms including granules, powders, tablets, capsules, syrups, emulsions, suspensions, and forms used for non-oral administration, for instance injections, sprays, transdermal patches or suppositories. These pharmaceutical forms can be prepared via known conventional techniques.

The preparation of an orally administered solid pharmaceutical form can be for instance performed by the following process: an excipient (for example lactose, sucrose, starch or mannitol), a desintegrant (for example calcium carbonate, calcium carboxymethylcellulose, alginic acid, sodium carboxymethylcellulose, colloidal silicon dioxide, sodium croscarmellose, crospovidone, guar gum, magnesium aluminium silicate, microcrystalline cellulose, cellulose powder, pregelatinised starch, sodium alginate or starch glycolate), a binder (for example alpha-starch, gum arabic, carboxymethylcellulose, polyvinylpyrrolidone, hydroxypropylcellulose, alginic acid, carbomer, dextrin, ethylcellulose, sodium alginate, maltodextrin, liquid glucose, magnesium aluminium silicate, hydroxyethylcellulose, methylcellulose or guar gum) and a lubricant (for example talc, magnesium stearate or polyethylene 6000) are added to the active principle and the mixture obtained is then tabletted. If necessary, the tablet can be coated via the known techniques, in order to mask the taste (for example with cocoa powder, mint, borneol or cinnamon powder) or to allow enteric dissolution or sustained release of the active principles. Coating products that can be used are, for example, ethylcellulose, hydroxymethylcellulose,

polyoxyethylene glycol, cellulose acetophthalate, hydroxypropylmethylcellulose phthalate and Eudragit® (methacrylic acid-acrylic acid copolymer), Opadry® (hydroxypropylmethylcellulose + macrogol + titanium oxide + lactose monohydrate). Pharmaceutically acceptable colorants may be added (for example yellow iron oxide, red iron oxide or quinoline yellow lake).

Liquid pharmaceutical forms for oral administration include solutions, suspensions and emulsions. The aqueous solutions can be obtained by dissolving the active principle in water, followed by addition of flavourings, colorants, stabilisers and/or thickeners, if necessary. In order to improve the solubility, it is possible to add ethanol, propylene glycol or any other pharmaceutically acceptable non-aqueous solvent. The aqueous suspensions for oral use can be obtained by dispersing the finely divided active principle in water with a viscous product, such as a natural or synthetic gum or resin, methylcellulose or sodium carboxymethylcellulose.

The pharmaceutical forms for injection can be obtained, for example, by the following process. The active principle is dissolved, suspended or emulsified either in an aqueous medium (for example distilled water, physiological saline or Ringer's solution) or in an oily medium (for example olive oil, sesame seed oil, cottonseed oil, corn oil or propylene glycol), with a dispersant (for example Tween® 80, HCO® 60 (Nikko Chemicals), polyethylene glycol, carboxymethylcellulose or sodium alginate), a preserving agent (for example methyl p-hydroxybenzoate, propyl p-hydroxybenzoate, benzyl alcohol, chlorobutanol or phenol), an isotonicity agent (for example sodium chloride, glycerol, sorbitol or glucose) and optionally other additives, such as, if desired, a solubilizing agent (for example sodium salicylate or sodium acetate) or a stabilizer (for example human serum albumin).

Pharmaceutical forms for external use (topical use) can be obtained from a solid, semi-solid or liquid composition containing the active principle. For example, to obtain a solid form, the active principle can be treated with excipients (for example lactose, mannitol, starch, microcrystalline cellulose or sucrose) and a thickener (for example natural gums, cellulose derivatives or acrylic polymers) so as to convert them into powder. The liquid pharmaceutical compositions are prepared in substantially the same way as the forms for injection, as indicated previously. The semi-solid pharmaceutical forms are preferably in the form of aqueous or oily gels or in the form of pomades. These compositions may optionally contain a pH regulator (for example carbonic acid, phosphoric acid, citric acid, hydrochloric acid or sodium hydroxide) and a preserving agent (for example a p-hydroxybenzoic acid ester, chlorobutanol or benzalkonium chloride).

A method for the treatment of a nervous and/or respiratory failure due to intoxication with at least one organophosphorous nerve agent, comprising administering at least one compound according to the invention is also described herein.

A method for the treatment of a neurological disease such as Alzheimer's disease, comprising administering at least one compound according to the invention is also described herein.

A method for the treatment of a cancer, comprising administering at least one compound according to the invention is also described herein.

A method for the treatment of a virus, comprising administering at least one compound according to the invention is also described herein.

Within the context of the invention, the term treatment denotes curative, symptomatic, and/or preventive treatments. In particular, it can refer to reducing the progression of the disease, reducing or suppressing at least one of its symptoms or complications, or improving in any way the state of health of patients.

The administration of the compounds or of the composition according to the invention may be performed before, during or after the exposition of the subject to the organophosphorous nerve agent.

In the present invention, the terms "subject" and "patient" are used indifferently and designate a human subject.

The amount of compound according to the invention to be administered according to the invention may vary in a broad range depending upon the patient, the mode of administration and the expected effect. In particular, the amount of compound according to the invention may be comprised between 200 mg and 4000 mg, with up to 3 daily intakes.

The compound or composition according to the invention may be co-administered with at least one other active agent, such as an antimuscarinic agent, in particular atropine, an anticonvulsant, in particular diazepam or one of its prodrugs, such as avizafone, and/or a bioscavenger able to capture and/or degrade OPNAs in blood, such as human butyrylcholinesterase.

The term co-administered means that the administration of the compound or composition according to the invention and that of the other active agent can be simultaneous, sequential and/or separate.

Other uses of the compounds of the invention

The compounds of this invention may further be used as tools for *in vivo* and/or *in vitro* biological studies. In this application, the compounds according to the invention may include one or more isotopes, which will allow for their detection.

The following examples are provided as illustrative, and not limitative, of the present invention.

## Examples

### Example 1: synthesis of compounds of the invention

**2-(2-(pyridin-3-yl)ethyl)thiazole-4-carbaldehyde oxime hydrochloride NM-55:**

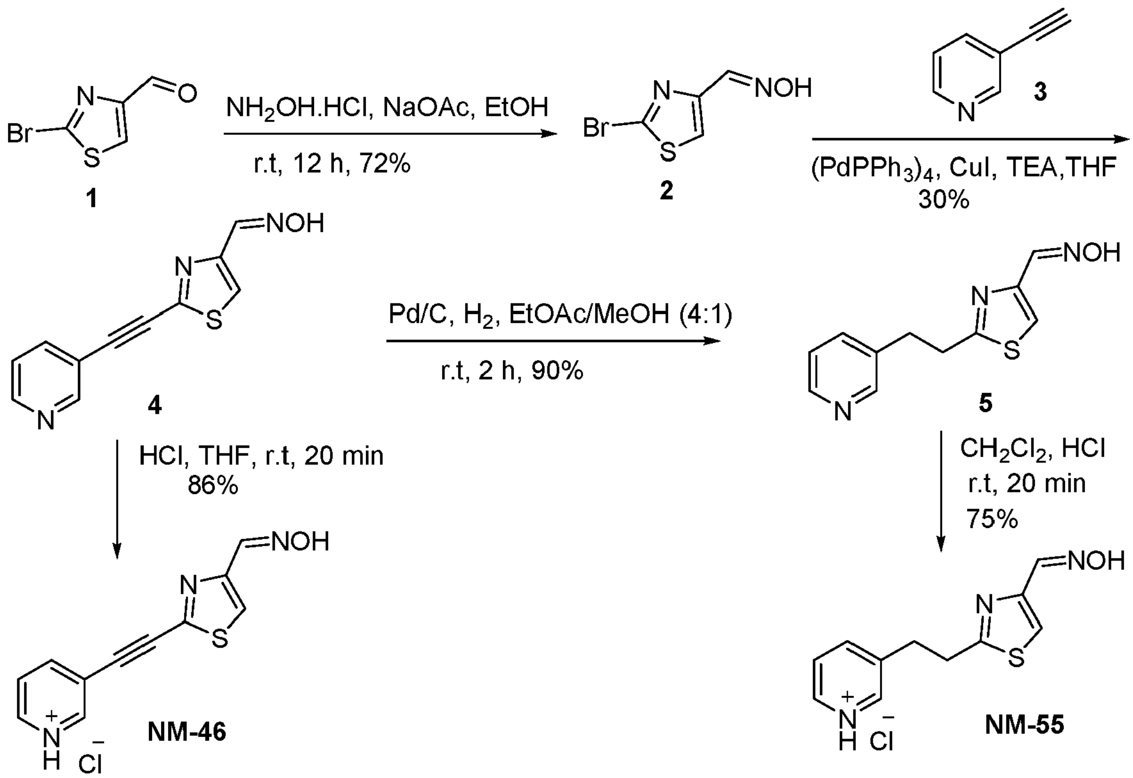


2-bromothiazole-4-carbaldehyde oxime (2)

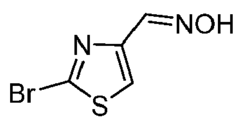

**2**

A solution of commercially available 2-bromothiazole-4-carbaldehyde 1 (200 mg, 1.04 mmol, 1equiv), hydroxylamine hydrochloride (144 mg, 2.04 mmol, 2 equiv), and NaOAc (256 mg, 3.12 mmol, 3 equiv) in EtOH (6 mL) was stirred at room temperature for 12 h. Upon completion, the reaction mixture was filtered through a small celite pad. The filtrate was concentrated under reduced pressure and the residue was purified by column chromatography (EtOAc/PE 1:9) to afford cis/trans isomers (1/0.56 ratio) of (E/Z)-2-bromothiazole-4-carbaldehyde oxime **2** as off white solid (155 mg, 72%); $R_f$ (20% EtOAc/PE) 0.50; NMR (400 MHz, $CDCl_3$): δ ppm 8.37 (s, 0.56 H), 8.18 (s, 1H), 7.73 (s, 0.56 H), 7.49(s, 1H); $^{13}C$ NMR (101 MHz, $CDCl_3$): δ ppm 148.78, 145.45, 143.45, 140.09, 137.26, 135.63, 129.52, 121.89.

**2-(pyridin-3-ylethynyl)thiazole-4-carbaldehyde oxime 4:**

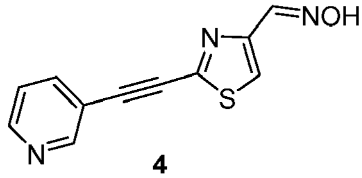

**4**

To a degassed solution of (E/Z)-2-bromothiazole-4-carbaldehyde oxime **2** (132 mg, 0.637 mmol, 1.1 equiv) in $THF/Et_3N$ (6 mL/ 2 mL), $Pd[PPh_3]_4$ (101 mg, 0.087 mmol, 0.15 equiv) and CuI (33 mg, 0.173mmol, 0.3 equiv) were added. After degassing the reaction mixture for 5 min at room temperature, 3-ethynylpyridine **3** (60 mg, 0.582mmol, 1 equiv) was added dropwise and the reaction mixture was stirred at room temperature for 16 h. Upon completion, the reaction mixture was concentrated under reduced pressure and the residue was purified by column chromatography (EtOAc/PE 50:50) to afford cis/trans isomers (7.5/2.5 ratio) of (E/Z)-2-(pyridin-3-ylethynyl)thiazole-4-carbaldehyde oxime **4** as a light yellowish solid (30 mg, 30%); IR (neat): $v_{max}$ 3081, 2784, 1478, 1405, 1281, 1104, 969, 778, 744, 696, 635, 569, 522, 486 $cm^{-1}$; $R_f$ (40% EtOAc/PE) 0.30; HRMS ($ESI^+$): *m/z* calcd for $C_{11}H_aN_3OS^+$ 230.038586 found 230.03829; $^1H$ NMR (400 MHz, $CDCl_3$): δ ppm 8.88-8.81 (m, 1H), 8.64 (dt, J = 9.0, 4.4 Hz, 1H), 8.52 (s, 0.75H), 8.27 (d, *J* = 8.8 Hz, 0.25H), 7.91 (tt, *J* = 7.7, 1.8 Hz, 1H), 7.81 (s, 0.75H), 7.65 (s, 0.25H), 7.40-7.33 (m, 1H). $^{13}C$ NMR (101 MHz, $CDCl_3$): δ ppm 152.36, 152.32, 149.81, 149.78, 149.75, 149.36, 148.42, 146.66, 146.08, 140.26, 139.58, 139.07, 139.04, 127.15, 123.31, 119.70, 118.58, 118.53, 90.93, 90.88, 84.95, 84.70.

**2-(pyridin-3-ylethynyl)thiazole-4-carbaldehyde oxime hydrochloride NM-46:**

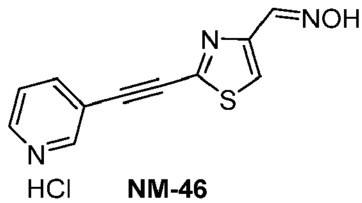

**NM-46**

To a solution of (E/Z)-2-(pyridin-3-ylethynyl)thiazole-4-carbaldehyde oxime **4** (12 mg, 0.052 mmol) in water (2 mL) was added 2N HCl (1 mL) at room temperature and stirred for 20 min at same temperature. Upon completion, solvent was distilled off under reduced pressure and the resulting solid was washed with diethyl ether (2 x 3 mL). The solid was dried under vacuum to give cis/trans isomers (3/2 ratio) of (E/Z)-2-(pyridin-3-ylethynyl)thiazole-4-carbaldehyde oxime hydrochloride **NM-46** as a pale yellow solid (12 mg, 86%); IR (neat): $v_{max}$ 3075, 2359, 1530, 955, 888, 798, 761, 667 $cm^{-1}$; HRMS ($ESI^+$): *m/z* calcd for $C_{11}H_9ClN_3OS^+$ 266.014937 found 266.013282; $^1H$ NMR (500 MHz, DMSO-$d_6$): δ ppm 12.22 (s, 0.4H), 11.50 (s, 0.6H), 9.22 (s, 1H), 8.79 (s, 1H), 8.74 (s, 0.4H), 8.25 (s, 0.6H), 8.16 (s, 0.6H), 8.13-8.10 (m, 1H), 7.80 (m, 1H), 7.75 (s, 0.4H); $^{13}C$ NMR (126 MHz, DMSO-de): δ ppm 161.94, 160.30, 149.85, 147.73, 145.70, 144.95, 143.28, 139.85, 138.24, 134.07, 131.23, 130.98, 127.63, 125.65, 123.74, 123.56, 121.08.

**2-(2-(pyridin-3-yl)ethyl)thiazole-4-carbaldehyde oxime 5:**

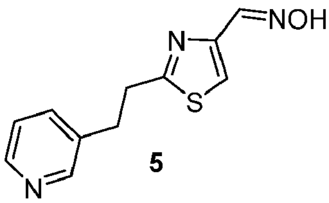

**5**

To a solution of (E/Z)-2-(pyridin-3-ylethynyl)thiazole-4-carbaldehyde oxime **4** (20 mg, 0.087 mmol) in 4:1 ratio of EtOAc/MeOH (5 mL) was added 10% Pd/C (10 mg) at room temperature under Argon atmosphere and stirred the mixture for 1 h under $H_2$ atmosphere using balloon pressure. Upon completion, the mixture was filtered through a small celite pad and concentrated under reduced pressure. The crude was purified by column chromatography (EtOAc/PE,

70:30) to afford cis/trans isomers (6/4 ratio) of (E/Z)-2-(2-(pyridin-3-yl)ethyl)thiazole-4-carbaldehyde oxime **5** as a white solid (17 mg, 83%); $R_f$ (50% EtOAc/PE) 0.20; IR (neat): $v_{max}$ 2921, 2850, 2360, 1731, 1579, 1421, 1123, 969, 913, 794, 704 $cm^{-1}$; HRMS (ESI$^+$): *m/z* calcd for $C_{11}H_{12}N_3OS^+$ 234.069559 found 234.068309; $^1H$ NMR (400 MHz, $CDCl_3$): δ ppm 8.50 (bs, 2H), 8.22 (s, 0.4H), 8.12 (s, 0.6H), 7.74 (s, 0.6H), 7.54 (d, *J* = 7.8 Hz, 1H), 7.37 (s, 0.4H), 7.27-7.18 (m, 1H), 3.40-3.28 (m, 2H), 3.18-3.14 (m, 2H); $^{13}C$ NMR (101 MHz, $CDCl_3$): δ 170.08, 168.13, 149.73, 149.70, 148.64, 147.83, 147.74, 144.21, 136.23, 135.64, 135.44, 124.16, 123.61, 118.38, 34.63, 34.25, 32.84, 32.71.

**2-(2-(pyridin-3-yl)ethyl)thiazole-4-carbaldehyde oxime hydrochloride NM-55:**

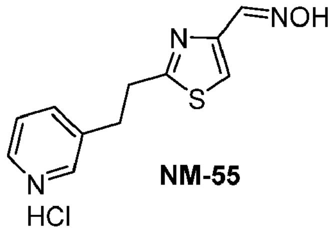

**NM-55**

To a solution of (E/Z)-2-(2-(pyridin-3-yl)ethyl)thiazole-4-carbaldehyde oxime **5** (14 mg, 0.06 mmol) in $CH_2Cl_2$ (3 ml) was added 2N HCl (0.1 mL) at room temperature and stirred for 30 min at same temperature. Upon completion, solvent was distilled off under reduced pressure and the resulting solid was washed with diethyl ether (2 x 3 mL). The solid was dried under vacuum to give cis/trans isomers (6.5/3.5 ratio) of (E/Z)-2-(2-(pyridin-3-yl)ethyl)thiazole-4-carbaldehyde oxime hydrochloride **NM-55** as a light brown solid (12 mg, 74%); IR (neat): $v_{max}$ 3056, 2360, 1632, 1556, 1470, 1263, 984, 926, 775, 679 $cm^{-1}$; $^1H$ NMR (400 MHz, $CD_3OD$): δ ppm 8.94 (s, 0.65H), 8.90 (s, 0.35H), 8.86-8.74 (m, 1H), 8.67 (t, *J* = 9.1 Hz, 1H), 8.55 (s, 0.35H), 8.22 (s, 1.7H), 7.96 (s, 0.65H), 7.65 (s, 0.35H), 3.75-3.59 (m, 2H), 3.48 (t, *J* = 7.3 Hz, 2H). $^{13}C$ NMR (101 MHz, $CD_3OD$): δ ppm 169.64, 147.20, 141.36, 141.31, 140.73, 140.26, 139.75, 139.59, 136.98, 127.21, 127.13, 125.72, 31.52, 31.35, 31.32.

**2-(4-(pyridin-3-yl)butyl)thiazole-4-carbaldehyde oxime hydrogen chloride NM-131: 2-(4-(pyridin-3-yl)but-1-yn-1-yl)thiazole-4-carbaldehyde oxime hydrogen chloride NM-132:**

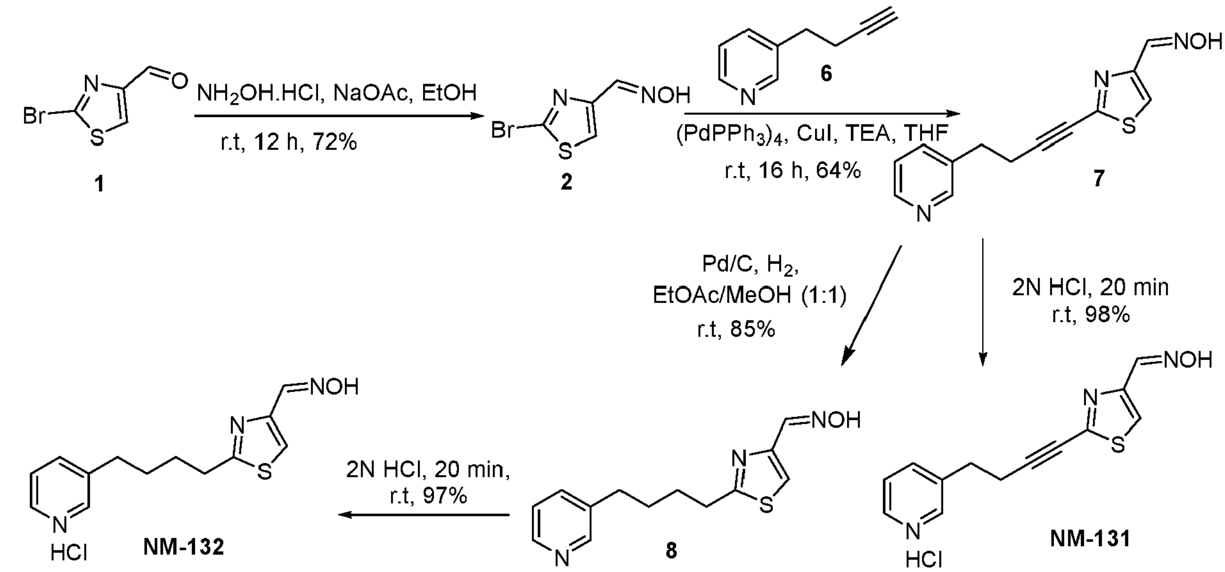


1 2 6 7 8 NM-132 NM-131

**2-(4-(pyridin-3-yl)but-1-yn-1-yl)thiazole-4-carbaldehyde oxime 7:**

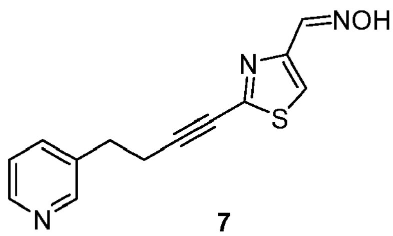

**7**

To a degassed solution of (E/Z)-2-bromothiazole-4-carbaldehyde oxime **2** (104 mg, 0.502 mmol, 1.1 equiv) in THF/$Et_3N$ (6 mL/ 2 mL), Pd[$PPh_3$]$_4$ (79.4 mg, 0.068 mmol, 0.15 equiv) and CuI (26 mg, 0.137 mmol, 0.3 equiv) were added. After degassing the reaction mixture for 5 min at room temperature, 3-(but-3-yn-1-yl)pyridine **6** (60 mg, 0.458 mmol, 1 equiv) was added dropwise and the reaction mixture was stirred at the room temperature for 16 h. Upon completion, the reaction mixture was concentrated under reduced pressure and the residue was purified by column chromatography (EtOAc/PE 70:30) to afford cis/trans isomers (8/2 ratio) of (E)-2-(4-(pyridin-3-yl)but-1-yn-1-yl)thiazole-4-carbaldehyde oxime **7** as an off white solid (75 mg, 64%); $R_f$ (40 % EtOAc/PE) 0.50; IR (neat): $v_{max}$ 3069, 2689, 2234, 1580, 1447, 1428, 1205, 1032, 969, 798, 772, 709, 643 $cm^{-1}$; HRMS ($ESI^+$): *m/z* calcd for $C_{13}H_{12}N_3OS^+$ 258.069559 found 258.067414; $^1H$ NMR (400 MHz, $CD_3OD$): δ ppm 8.50 (bs, 3H), 7.84 (d, *J* = 7.8 Hz, 1H), 7.72 (s, 0.2H) 7.55 (s, 0.8H), 7.44 (bs, 1H), 3.00 (t, *J* = 6.9 Hz, 2H), 2.86 (t, *J* = 6.9 Hz, 2H); $^{13}C$ NMR (101 MHz, $CD_3OD$): δ ppm 150.27, 149.12, 148.20, 146.75, 140.21, 138.48, 127.49, 96.98, 96.75, 75.45, 32.10, 21.79.

As a note, 3-(but-3-yn-1-yl)pyridine **6** may be described as in Galli et al, Chem Med Chem 2008, 3, 771-779; or Joseph et al, J. Org. Chem. 2013, 78, 1670-1676.

**2-(4-(pyridin-3-yl)but-1-yn-1-yl)thiazole-4-carbaldehyde oxime hydrochloride NM-131:**

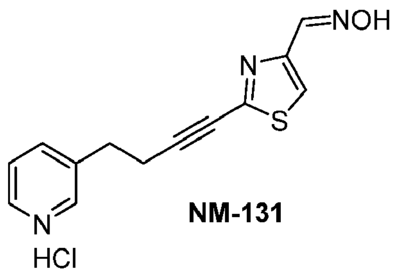

**NM-131**

To a solution of (E/Z)-2-(4-(pyridin-3-yl)but-1-yn-1-yl)thiazole-4-carbaldehyde oxime **7** (16 mg, 0.062 mmol) was added 2N HCl (2 mL) at room temperature and stirred for 20 min at same temperature. Upon completion, solvent was distilled off under reduced pressure and the resulting solid was washed with diethyl ether (2 x 4 mL). The solid was dried under vacuum to give cis/trans mixture (6.5/3.5 ratio) of (E/Z)-2-(4-(pyridin-3-yl)but-1-yn-1-yl)thiazole-4-carbaldehyde oxime hydrochloride **NM-131** as a light brown solid (18 mg, 98%); IR (neat): $v_{max}$ 3037, 2361, 1632, 1544, 1463, 979, 912, 792, 771, 678, 618 $cm^{-1}$; HRMS ($ESI^+$): *m/z* calcd for $C_{13}H_{13}ClN_3OS^+$ 294.046237 found 294.04566; $^1H$ NMR (400 MHz, $CD_3OD$): δ ppm 8.91-8.61 (m, 4H), 8.08 (s, 1H), 7.64-7.56 (m, 0.35H), 7.23-6.91 (m, 0.65H), 3.32-3.02 (m, 4H); $^{13}C$ NMR (101 MHz, $CD_3OD$): δ ppm 162.40, 149.17, 148.96, 143.42, 143.13, 142.22, 142.13, 141.53, 141.35, 140.90, 129.11, 128.90, 127.12, 122.27, 42.42, 41.89, 31.74, 31.65.

**2-(4-(pyridin-3-yl)butyl)thiazole-4-carbaldehyde oxime 8:**

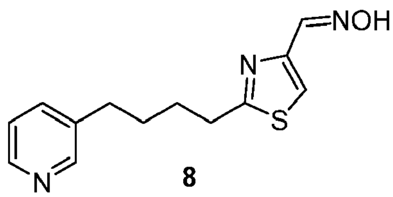

**8**

To a solution of (E/Z)-2-(4-(pyridin-3-yl)but-1-yn-1-yl)thiazole-4-carbaldehyde oxime **7** (20 mg, 0.077 mmol) in 4:1 ratio of EtOAc/MeOH (5 mL) was added 10% Pd/C (10 mg) at room temperature under Argon atmosphere and stirred the mixture for 2 h under $H_2$ atmosphere using balloon pressure. Upon completion, the mixture was filtered through a small celite pad and concentrated under reduced pressure. The crude was purified by preparative TLC (EtOAc/PE, 1:1) to afford cis/trans isomers (1/1 ratio) of (E/Z)-2-(4-(pyridin-3-yl)butyl)thiazole-4-carbaldehyde oxime **8** as white solid (17.6 mg, 83%); $R_f$ (60% EtOAc/PE) 0.20; IR (neat): $v_{max}$ 2922, 2854, 2360, 2340, 1579, 1462, 1424, 1098, 967, 924, 796, 781, 706, 642 $cm^{-1}$; HRMS (ESI$^+$): *m/z* calcd for $C_{13}H_{16}N_3OS^+$ 262.100860 found 260.100806; $^1H$ NMR (400 MHz, $CD_3OD$): δ ppm 8.34-8.22 (m, 2.5H), 8.02 (s, 0.5H) 7.60 (d, *J* = 7.8 Hz, 1H), 7.47 (d, *J* = 9.1 Hz, 1H), 7.25 (dd, *J* = 7.4, 4.9 Hz, 1H), 2.96 (t, *J* = 7.4 Hz, 2H), 2.62 (t, *J* = 6.7 Hz, 2H), 1.80-1.50 (m, 4H); $^{13}C$ NMR (101 MHz, $CD_3OD$): δ ppm 173.92, 171.67, 150.04, 147.55, 146.20, 144.27, 140.67, 139.78, 138.28, 125.88, 125.21, 119.08, 33.57, 33.33, 33.30, 33.28, 31.44, 30.49, 30.44.

**2-(4-(pyridin-3-yl)butyl)thiazole-4-carbaldehyde oxime hydrochloride NM-132:**

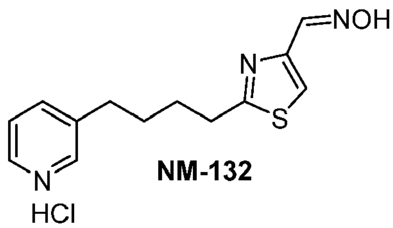

**NM-132**

To a solution of (E/Z)-2-(4-(pyridin-3-yl)butyl)thiazole-4-carbaldehyde oxime **8** (10 mg, 0.06 mmol) was added 2N HCl (2 mL) at room temperature and stirred for 20 min at same temperature. Upon completion, solvent was distilled off under reduced pressure and the resulting solid was washed with diethyl ether (2 x 3 mL). The solid was dried under vacuum to give cis/trans mixture (7.5/ 2.5 ratio) of (E/Z)-2-(4-(pyridin-3-yl)butyl)thiazole-4-carbaldehyde oxime hydrochloride **NM-132** as a light brown solid (11 mg, 97.3%); IR (neat): $v_{max}$ 3049, 1629, 1612, 1552, 1466,1381, 1261, 991, 793, 680 $cm^{-1}$; HRMS (ESI$^+$): *m/z* calcd for $C_{13}H_{16}N_3OS^+$ 262.100860 found 260.1004291; $^1H$ NMR (500 MHz, $CD_3OD$): δ ppm 9.02-8.50 (m, 3.5H), 8.37-78.94 (m, *J* = 2.25 H), 7.67 (s, 0.25H) 3.37 (s, 2H), 3.03 (s, 2H), 2.18-1.76 (m, 4H); $^{13}C$ NMR (126 MHz, $CD_3OD$): δ ppm 173.92, 171.67, 150.04, 147.55, 146.20, 144.27, 140.67, 139.78, 138.28, 125.88, 125.21, 119.08, 33.57, 33.33, 33.30, 33.28, 31.44, 30.49, 30.44.

**2-(2-(pyridin-2-yl)ethyl)thiazole-4-carbaldehyde oxime hydrochloride NM-109:**

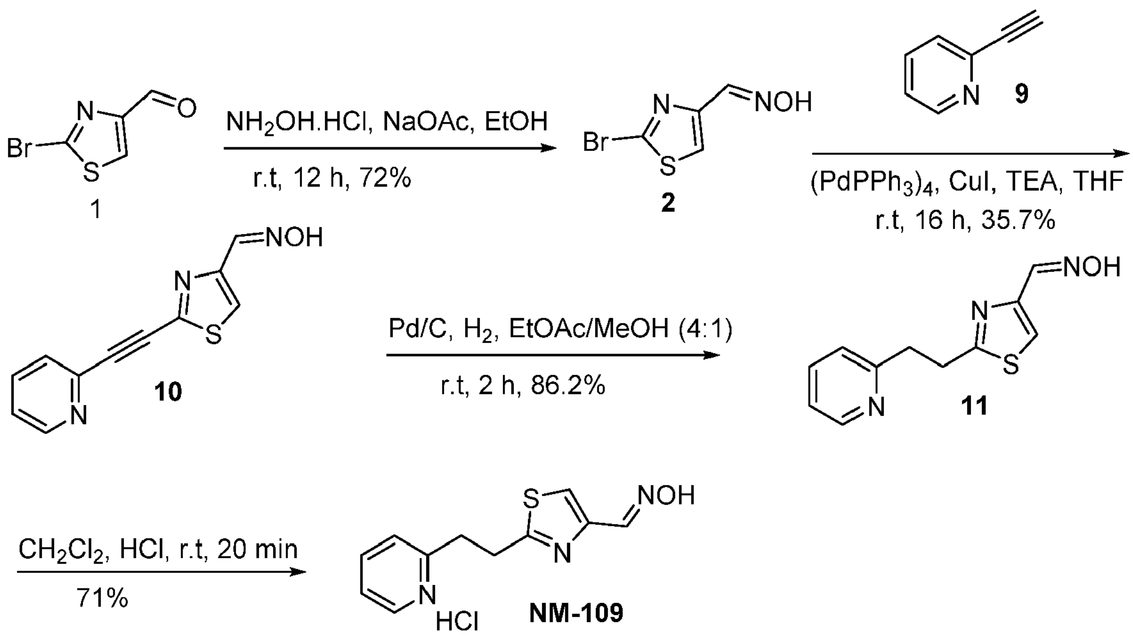


**2-(pyridin-2-ylethynyl)thiazole-4-carbaldehyde oxime 10:**

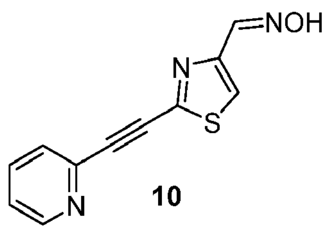

10

To a degassed solution of (E/Z)-2-bromothiazole-4-carbaldehyde oxime **2** (132 mg, 0.637 mmol, 1.1 equiv) in THF/$Et_3N$ (6 mL/ 2 mL), Pd[$PPh_3$]$_4$ (101 mg, 0.087 mmol, 0.15 equiv) and CuI (33 mg, 0.173 mmol, 0.3 equiv) were added. After degassing the reaction mixture for 5 min at room temperature, 2-ethynylpyridine **9** (60 mg, 0.582 mmol, 1 equiv) was added dropwise and the reaction mixture was stirred at the room temperature for 16 h. Upon completion, the reaction mixture was concentrated under reduced pressure and the residue was purified by column chromatography (EtOAc/PE 50:50) to afford (E)-2-(pyridin-2-ylethynyl)thiazole-4-carbaldehyde oxime **10** as an off white solid (40.5 mg, 35.7%); $R_f$ (40% EtOAc/PE) 0.25; IR (neat): $v_{max}$ 3055, 2850, 1581, 1465, 1435, 1275, 1110, 996, 979, 769, 720, 692, 537 cm$^{-1}$; HRMS (ESI$^+$): *m/z* calcd for $C_{11}H_8N_3OS^+$ 230.038259 found 230.038791; $^1$H NMR (400 MHz, $CD_3OD$): δ ppm 8.71 (s, 1H), 8.66-8.63 (m, 1H), 7.95 (td, *J* = 7.8, 1.7 Hz, 1H), 7.78 (d, *J* = 7.9 Hz, 1H), 7.67 (s, 1H), 7.53 (ddd, J = 7.7, 5.0, 1.1 Hz, 1H); $^{13}$C NMR (101 MHz, $CD_3OD$): δ ppm 151.25, 147.71, 147.57, 142.28, 140.18, 138.79, 129.43, 128.97, 125.97, 93.08, 81.94.

**2-(2-(pyridin-2-yl)ethyl)thiazole-4-carbaldehyde oxime 11:**

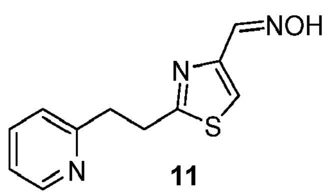

11

To a solution of (E)-2-(pyridin-2-ylethynyl)thiazole-4-carbaldehyde oxime **10** (40 mg, 0.087 mmol) in 4:1 ratio of EtOAc/MeOH (5 mL) was added 10% Pd/C (20 mg) at room temperature under Argon atmosphere and stirred the mixture for 2 h under $H_2$ atmosphere using balloon pressure. Upon completion, the mixture was filtered using celite pad and concentrated under reduced pressure. The crude was purified by column chromatography (EtOAc/PE, 70:30) to afford trans-cis isomers (6/4 ratio) of (E/Z)-2-(2-(pyridin-2-yl)ethyl)thiazole-4-carbaldehyde oxime **11** as pale yellow solid (35 mg, 86.2%); $R_f$ (50% EtOAc/PE) 0.20; IR (neat): $v_{max}$ 2920, 2851, 1592, 1569, 1476, 1435, 1174, 1117, 975, 922, 748, 721, 694, 539 $cm^{-1}$; HRMS ($ESI^+$): *m/z* calcd for $C_{11}H_{12}N_3OS^+$ 234.069559 found 234.069476.

**2-(2-(pyridin-2-yl)ethyl)thiazole-4-carbaldehyde oxime hydrochloride NM-109:**

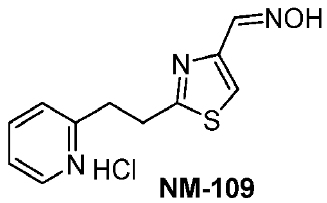

**NM-109**

To a solution of (E/Z)-2-(2-(pyridin-2-yl)ethyl)thiazole-4-carbaldehyde oxime **11** (25 mg, 0.06 mmol) in $CH_2Cl_2$ (3 mL) was added 2N HCl (0.5 mL) at room temperature and stirred for 30 min at same temperature. Upon completion, solvent was distilled off under reduced pressure and the resulting solid was washed with diethyl ether (2 x 3 mL). The solid was dried under vacuum to give cis/trans isomer (6.5/3.5 ratio) of (E/Z)-2-(2-(pyridin-2-yl)ethyl)thiazole-4-carbaldehyde oxime hydrochloride **NM-109** as a light brown solid (20 mg, 71%); IR (neat): $v_{max}$ 3055, 2922, 1617, 1435, 1189, 1117, 996, 752, 718, 692, 536 $cm^{-1}$; HRMS ($ESI^+$): *m/z* calcd for $C_{11}H_{12}N_3OS^+$ 234.0696 found 234.0697; $^1H$ NMR (400 MHz, $CD_3OD$): δ ppm 8.82-8.79 (m, 1H), 8.61-8.56 (m, 1H), 8.47 (s, 0.3H), 8.17 (s, 0.7H), 8.09 (dd, *J* = 8.1, 4.9 Hz, 1H), 8.00 (d, *J* = 6.4 Hz, 1H), 7.80 (s, 0.7H), 7.59 (s, 0.3H), 3.68 (m, 4H); $^{13}C$ NMR (101 MHz, $CD_3OD$): δ ppm 168.63, 156.79, 156.41, 148.23, 148.19, 145.46, 142.93, 142.49, 142.41, 139.86, 128.93, 126.90, 126.67, 126.60, 121.13, 33.41, 33.27, 31.72.

**(E/Z)-2-(2-(pyridin-4-yl)ethyl)thiazole-4-carbaldehyde oxime hydrochloride NM-130:**

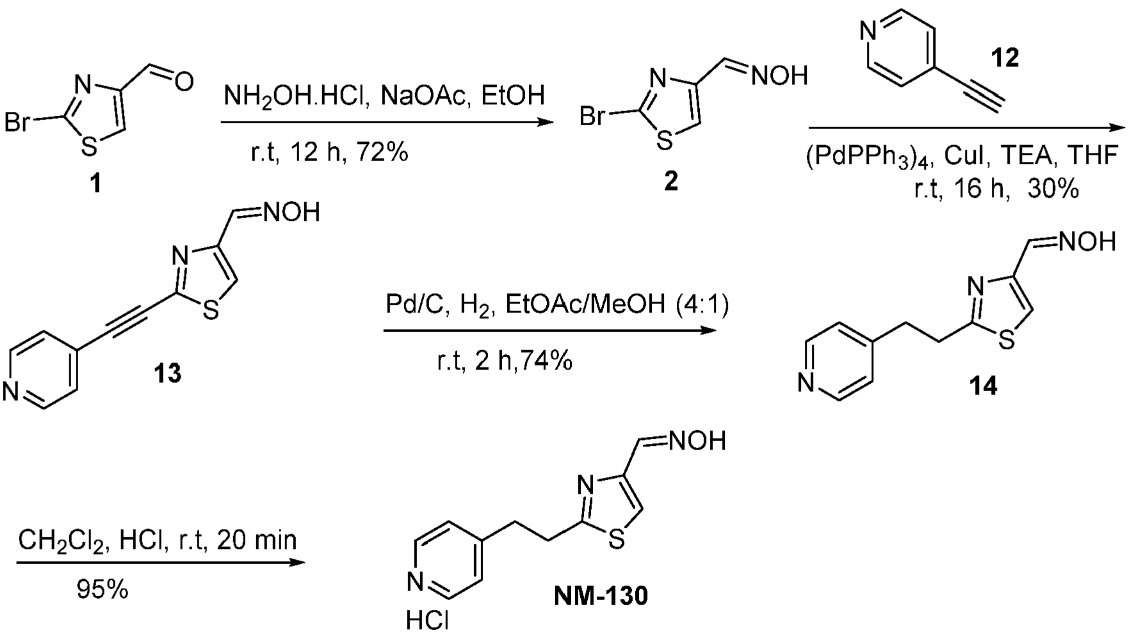

**2-(pyridin-4-ylethynyl)thiazole-4-carbaldehyde oxime 13:**

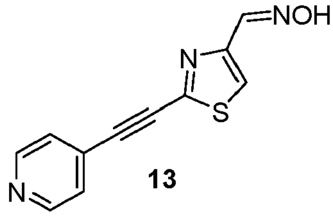

**13**

To a degassed solution of (E/Z)-2-bromothiazole-4-carbaldehyde oxime **2** (132 mg, 0.637 mmol, 1.1 equiv) in THF/$Et_3N$ (6 mL/ 2 mL), Pd[$PPh_3$]$_4$ (101 mg, 0.087 mmol, 0.15 equiv) and CuI (33 mg, 0.173 mmol, 0.3 equiv) were added. After degassing the reaction mixture for 5 min at room temperature, 4-ethynylpyridine **12** (60 mg, 0.582 mmol, 1 equiv) was added dropwise and the reaction mixture was stirred at the room temperature for 16 h. Upon completion, the reaction mixture was concentrated under reduced pressure and the residue was purified by column chromatography (EtOAc/PE 50:50) to afford cis/trans isomer (6/4 ratio) of (E/Z)-2-(pyridin-4-ylethynyl)thiazole-4-carbaldehyde oxime **13** as off white solid (30 mg, 30%); $R_f$ (50% EtOAc/PE) 0.25; IR (neat): $v_{max}$ 3062, 2715, 2359, 1569, 1497, 1405, 1322, 1099, 994, 978, 925, 820, 701, 571, 542 $cm^{-1}$; HRMS (ESI$^+$): *m/z* calcd for $C_{11}H_8N_3OS^+$ 230.038259 found 230.038473; $^1$H NMR (400 MHz, DMSO-$d_6$): δ ppm 12.27 (s, 0.6H), 11.56 (s, 0.4H), 8.73-8.71 (m, 2.6H), 8.24 (s, 0.4H), 8.14 (s, 0.4H), 7.75 (s, 0.6H), 7.69-7.62 (m, 2H); $^{13}$C NMR (101 MHz, DMSO-de): δ ppm 150.19, 146.61, 146.01, 145.21, 142.64, 139.00, 131.55, 131.45, 128.84, 128.73, 128.62, 128.19, 121.90, 90.67, 90.63, 85.50, 85.35.

**2-(2-(pyridin-4-yl)ethyl)thiazole-4-carbaldehyde oxime 14:**

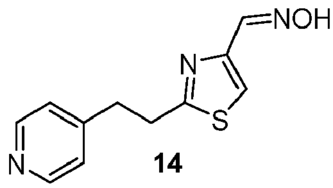

**14**

To a solution of (E/Z)-2-(pyridin-4-ylethynyl)thiazole-4-carbaldehyde oxime **13** (20 mg, 0.087 mmol) in 1:1 ratio of EtOAc/MeOH (5 mL) was added 10% Pd/C (20 mg) at room temperature under Argon atmosphere and stirred the mixture for 1 h under $H_2$ atmosphere using balloon pressure. Upon completion, the mixture was filtered using celite pad and concentrated under reduced pressure. the crude was purified by column chromatography (EtOAc/PE, 80:20) to afford cis/trans isomers (5.5/4.5 ratio) of (E/Z)-2-(2-(pyridin-4-yl)ethyl)thiazole-4-carbaldehyde oxime **14** as off white solid (15 mg, 74%); $R_f$ (100% EtOAc) 0.25; IR (neat) $v_{max}$ 2922, 2852, 2359, 1605, 1419, 1189, 1111, 969, 920, 853, 805, 751, 721, 540, 502 $cm^{-1}$; HRMS (ESI$^+$): *m/z* calcd for $C_{11}H_{12}N_3OS^+$ 234.069559 found 234.069628; $^1$HNMR (400 MHz, $CD_3OD$): δ ppm 8.31 (bs, 2H), 8.28 (s, 0.45H), 8.02 (s, 0.55H), 7.48 (s, 0.55H), 7.47 (s, 0.45H), 7.22 (m, 2H), 3.28 (td, *J* = 7.6, 3.1 Hz, 2H), 3.08 (t, *J* = 7.6 Hz, 2H); $^{13}$C NMR (101 MHz, $CD_3OD$): δ ppm 171.98, 169.73, 152.18, 150.19, 150.02, 150.02, 146.36, 144.27, 140.70, 126.09, 125.73, 119.32, 35.80, 35.76, 34.19, 33.93.

**2-(2-(pyridin-4-yl)ethyl)thiazole-4-carbaldehyde oxime hydrochloride NM-130:**

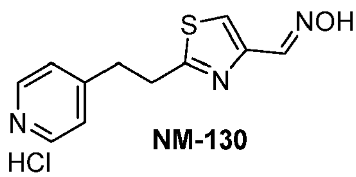

**NM-130**

To a solution of (E/Z)-2-(2-(pyridin-4-yl)ethyl)thiazole-4-carbaldehyde oxime **14** (15 mg, 0.064 mmol) was added

2N HCl (2 mL) at room temperature and stirred for 30 min at same temperature. Upon completion, solvent was distilled off under reduced pressure and the resulting solid was washed with diethyl ether (2 x 4 mL). The solid was dried under vacuum to give cis/trans isomers (7/3 ratio) of (E/Z)-2-(2-(pyridin-4-yl)ethyl)thiazole-4-carbaldehyde oxime hydrochloride **NM-130** as a light brown solid (16.5 mg, 95%); IR (neat) $v_{max}$ 3298, 3107, 2989, 2599, 1635, 1304, 1436, 1011, 888, 849, 809, 785, 542 $cm^{-1}$; HRMS (ESI$^+$): *m/z* calcd for $C_{11}H_{12}N_3OS^+$ 234.069559 found 234.069336; $^1H$ NMR (400 MHz, $CD_3OD$): δ ppm 8.82 (m, 2H), 8.56 (s, 0.3H), 8.21 (s, 0.7H), 8.11 (m, 2H), 7.97 (s, 0.7H), 7.65 (s, 0.3H), 3.82-3.63 (m, 2H), 3.57 (m, 2H); $^{13}C$ NMR (101 MHz, $CD_3OD$): δ ppm 169.68, 162.07, 161.45, 141.16, 141.03, 139.55, 139.53, 136.84, 127.51, 125.85, 120.39, 120.32, 34.57, 34.52, 30.69, 30.35.

**2-(2-(pyridin-3-yl)ethyl)thiazole-5-carbaldehyde oxime hydrogen chloride NM-139:**

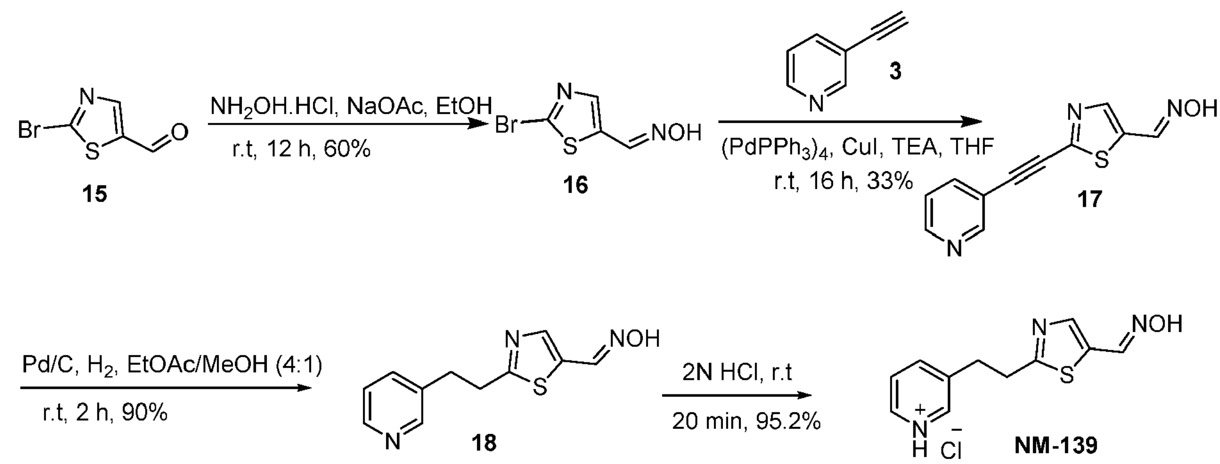

15 16 17 18 NM-139

**(E/Z)-2-bromothiazole-4-carbaldehyde oxime 16:**

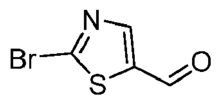

**16**

A solution of commercially available 2-bromothiazole-5-carbaldehyde **15** (500 mg, 2.60 mmol, 1equiv), hydroxylamine hydrochloride (361mg, 5.19mmol, 2 equiv), and $CH_3CO_2Na$ (640 mg, 7.80 mmol, 3 equiv) in EtOH (15 mL) was stirred at 80 °C for 12 h. Upon completion, the reaction mixture was filtered through a small celite pad. The filtrate was concentrated under reduced pressure and the residue was purified by column chromatography (EtOAc/PE 1:9) to afford cis/trans isomers (1/0.7 ratio) of (E/Z)-2-bromothiazole-5-carbaldehyde oxime **16** as off white solid (330 mg, 61.2%); $R_f$ (20% EtOAc/PE) 0.45; $^1H$ NMR (400 MHz, DMSO-de): δ ppm 12.50 (s, 1H), 11.68 (s, 0.7H), 8.38 (s, 0.7H), 8.08 (s, 1H), 8.00 (s, 1H), 7.85 (s, 0.7H); $^{13}C$ NMR (101 MHz, DMSO-$d_6$): δ ppm 145.81, 143.85, 141.60, 141.28, 137.98, 136.72, 136.69, 129.34.

**2-(pyridin-3-ylethynyl)thiazole-5-carbaldehyde oxime 17:**

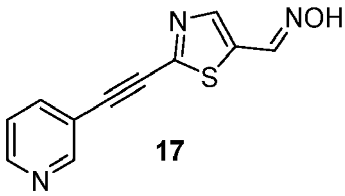

17

To a degassed solution of (E/Z)-2-bromothiazole-5-carbaldehyde oxime **16** (165.8 mg, 0.80 mmol, 1.1 equiv) in THF/$Et_3N$ (6 mL/ 2 mL), Pd[$PPh_3$]$_4$ (126.3 mg, 0.109 mmol, 0.15 equiv) and CuI (41.6 mg, 0.218 mmol, 0.3 equiv) were added. After degassing the reaction mixture for 5 min at room temperature, 3-ethynylpyridine **3** (75 mg, 0.728 mmol, 1 equiv) was added dropwise and the reaction mixture was stirred at room temperature for 16 h. Upon completion, the reaction mixture was concentrated under reduced pressure and the residue was purified by column chromatography (EtOAc/PE 50:50) to afford cis/trans isomers (9/1 ratio) of (E/Z)-2-(pyridin-3-ylethynyl)thiazole-5-carbaldehyde oxime **17** as a pale yellow solid (55 mg, 33%); $R_f$ (50% EtOAc/PE) 0.25; IR (neat): $v_{max}$ 2665, 2358, 1482, 1410, 1266, 1212, 1110, 927, 889, 839, 805, 692, 625, 590 $cm^{-1}$; HRMS (ESI$^+$): *m/z* calcd for $C_{11}H_8N_3OS^+$ 230.038259 found 230.037096; $^1H$ NMR (400 MHz, DMSO-de): δ ppm 12.64 (s, 0.9H), 11.78 (s, 0.1H), 8.88 (s, 1H), 8.70 (s, 1H), 8.45 (s, 0.1H), 8.36 (s, 1H), 8.17-8.05 (m, 2H), 7.54 (dd, *J* = 7.8, 4.7 Hz, 1H); $^{13}C$ NMR (101 MHz, DMSO-de): δ ppm 152.42, 150.69, 149.92, 147.63, 139.59, 138.00, 135.19, 127.30, 124.29, 92.69, 85.55.

**2-(2-(pyridin-3-yl)ethyl)thiazole-5-carbaldehyde oxime 18:**

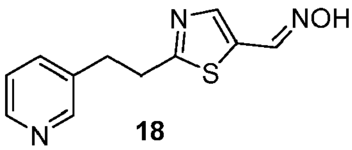

18

To a solution of (E/Z)-2-(pyridin-3-ylethynyl)thiazole-5-carbaldehyde oxime **17** (25 mg, 0.109 mmol) in 1:1 ratio of MeOH/THF (5 mL) was added 10% Pd/C (20 mg) at room temperature under Argon atmosphere and stirred the mixture for 2 h under $H_2$ atmosphere using balloon pressure. Upon completion, the mixture was filtered through a small celite pad and concentrated under reduced pressure and the crude was purified by column chromatography (EtOAc/PE, 70:30) to afford cis/trans isomers (9/1 ratio) of (E/Z)-2-(2-(pyridin-3-yl)ethyl)thiazole-5-carbaldehyde oxime **18** as white solid (23 mg, 90%); $R_f$ (100% EtOAc/PE) 0.25; IR (neat): $v_{max}$ 2996, 2348, 1578, 1480, 1416, 1187, 916, 884, 799, 704, 663, 609 $cm^{-1}$; HRMS (ESI$^+$): *m/z* calcd for $C_{11}H_{12}N_3OS^+$ 234.069559 found 234.070401; $^1H$ NMR (400 MHz, $CD_3OD$): δ ppm 8.39 (bs, 2H), 8.23 (s, 0.1H), 8.00 (s, 0.9H), 7.75 (s, 1H), 7.72 (d, *J* = 7.9 Hz, 1H), 7.36 (s, 1H), 3.37 (t, *J* = 7.7 Hz, 2H), 3.17 (t, J = 7.5 Hz, 2H); $^{13}C$ NMR (101 MHz, $CD_3OD$): δ ppm 173.88, 148.78, 146.67, 144.99, 142.28, 140.93, 137.54, 137.03, 125.71, 33.29, 32.25.

**2-(2-(pyridin-3-yl)ethyl)thiazole-5-carbaldehyde oxime hydrochloride NM-139:**

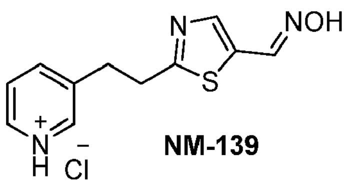

**NM-139**

To a solution of (E/Z)-2-(2-(pyridin-3-yl)ethyl)thiazole-4-carbaldehyde oxime **18** (20 mg, 0.06 mmol) in 2N HCl (2 mL) at room temperature and stirred for 20 min at same temperature. Upon completion, solvent was distilled off under reduced pressure and the resulting solid was washed with diethyl ether (2 x 4 mL). The solid was dried under vacuum to give cis/trans isomers (8/2 ratio) of (E/Z)-2-(2-(pyridin-3-yl)ethyl)thiazole-4-carbaldehyde oxime hydrochloride **NM-139** as a light brown solid (22 mg, 95.2%); IR (neat): $v_{max}$ 3047, 1555, 1468, 1216, 994, 928, 799, 734, 679, 626 $cm^{-1}$; HRMS (ESI$^+$): *m/z* calcd for $C_{11}H_{12}N_3OS^+$ 234.06959 found 234.069456; $^1H$ NMR (400 MHz, $CD_3OD$): δ ppm 8.95 (bs,

1H), 8.82 (d, *J* = 5.0 Hz, 1H), 8.69 (d, *J* = 7.2 Hz, 1H), 8.47 (s, 0.8H), 8.32 (s, 0.2H), 8.17-8.05 (m, 1.2H), 7.98 (s, 0.8H), 3.81-3.63 (m, 2H), 3.56-3.41 (m, 2H); $^{13}C$ NMR (101 MHz, $CD_3OD$): δ ppm 175.36, 148.62, 142.80, 141.65, 141.44, 141.29, 141.19, 141.01, 139.13, 138.08, 137.34, 128.71, 128.65, 128.52, 128.29, 33.15, 32.88, 32.67, 32.01.

**2-(4-(pyridin-3-yl)but-1-yn-1-yl)thiazole-5-carbaldehyde oxime hydrogen chloride NM-141:**

**2-(4-(pyridin-3-yl)butyl)thiazole-5-carbaldehyde oxime hydrogen chloride NM-143:**

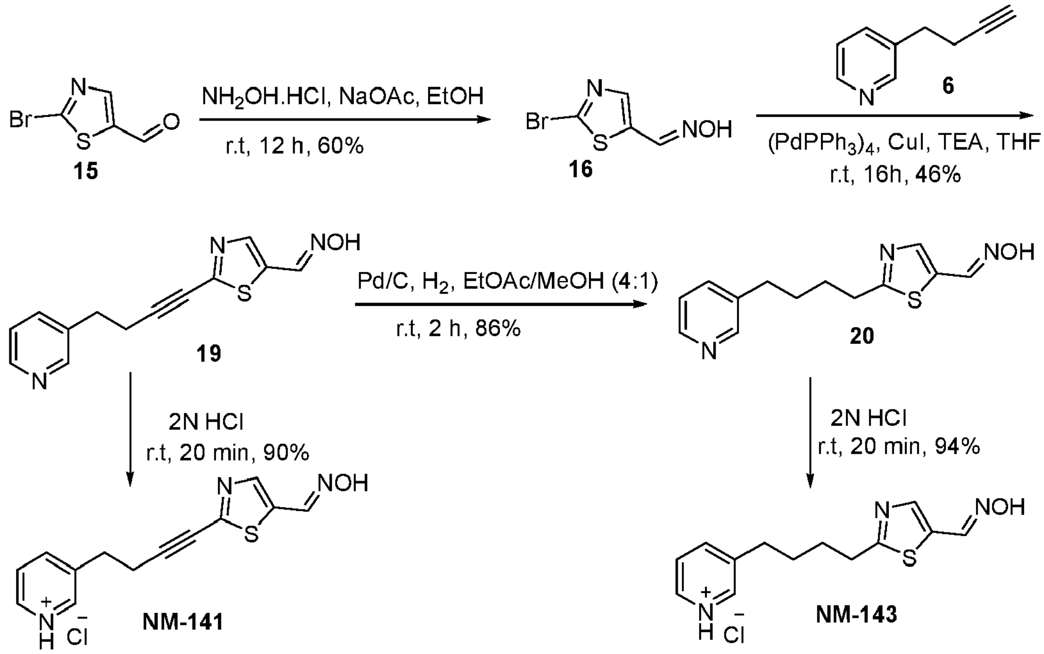


**2-(4-(pyridin-3-yl)but-1-yn-1-yl)thiazole-5-carbaldehyde oxime 19:**

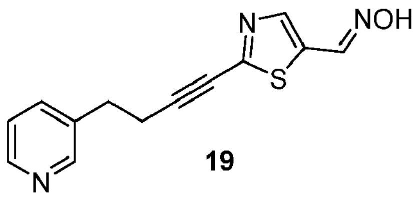

**19**

To a degassed solution of (E/Z)-2-bromothiazole-5-carbaldehyde oxime **16** (173 mg, 0.835 mmol, 1.1 equiv) in THF/$Et_3N$ (9 mL/ 3 mL), Pd[$PPh_3$]$_4$ (132 mg, 0.114 mmol, 0.15 equiv) and CuI (43.6 mg, 0.228 mmol, 0.3 equiv) were added. After degassing the reaction mixture for 5 min at room temperature, 3-(but-3-yn-1-yl)pyridine **6** (100 mg, 0.763 mmol, 1 equiv) was added dropwise and the reaction mixture was stirred at the room temperature for 16 h. Upon completion, the reaction mixture was concentrated under reduced pressure and the residue was purified by column chromatography (EtOAc 100%) to afford cis/trans isomers (95/5 ratio) of (E/Z)-2-(4-(pyridin-3-yl)but-1-yn-1-yl)thiazole-5-carbaldehyde oxime **19** as an off white solid (90 mg, 46%); $R_f$ (100% EtOAc) 0.25; IR (neat): $\nu_{max}$ 3357, 3160, 2360, 2340, 2224, 1651, 1605, 1411, 1264, 1139, 791, 711, 624 $cm^{-1}$; HRMS ($ESI^+$): *m/z* calcd for $C_{13}H_{12}N_3OS^+$ 258.069559 found 258.068945; $^1H$ NMR (400 MHz, DMSO-de): δ ppm 12.45 (s, 0.95H), 11.64 (s, 0.5H), 8.77-8.28 (m, 2H), 8.20 (s, 1H), 8.01 (s, 1H), 7.76 (d, *J* = 7.7 Hz, 1H), 7.44-7.32 (m, 1H), 2.91 (m, 4H); $^{13}C$ NMR (101 MHz, DMSO-de): δ ppm 151.17, 150.26, 148.25, 148.09, 148.09, 147.07, 138.00, 136.47, 126.15, 98.15, 75.42, 30.98, 21.02.

**2-(4-(pyridin-3-yl)but-1-yn-1-yl)thiazole-5-carbaldehyde oxime hydrochloride NM-141:**

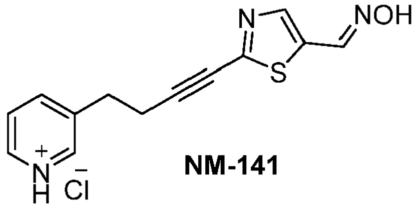

**NM-141**

To a solution of (E/Z)-2-(4-(pyridin-3-yl)but-1-yn-1-yl)thiazole-5-carbaldehyde oxime **19** (20 mg, 0.077 mmol) was added 2N HCl (2 mL) at room temperature and stirred for 20 min at same temperature. Upon completion, solvent was distilled off under reduced pressure and the resulting solid was washed with diethyl ether (2 x 4 mL). The solid was dried under vacuum to give cis/trans mixture isomers (7.5/2.5 ratio) of (E/Z)-2-(4-(pyridin-3-yl)but-1-yn-1-yl)thiazole-5-carbaldehyde oxime hydrochloride **NM-141** as a light brown solid (20.5 mg, 90%); IR (neat): $v_{max}$ 3004, 2555, 2360, 2341, 1633, 1558, 1263, 1156, 928, 802, 681 $cm^{-1}$; HRMS ($ESI^+$): *m/z* calcd for $C_{13}H_{13}ClN_3OS^+$ 294.046237 found 294.046258; $^1H$ NMR (400 MHz, $CD_3OD$): δ ppm 8.91 (bs, 1H), 8.78 (bs, 1H), 8.67 (d, *J* = 7.3 Hz, 1H), 8.47 (s, 0.75H), 8.34 (s, 2.5H), 8.12 (bs, 1H), 7.28 (bs, 0.75H), 7.12 (bs, 0.25), 341-3.34 (m, 4H), 3.21 (m, 4H); $^{13}C$ NMR (101 MHz, $CD_3OD$): δ ppm 147.30, 147.24, 145.24, 141.26, 140.58, 139.99, 139.69, 138.01, 127.26, 118.15, 40.39, 40.35, 30.04.

**2-(4-(pyridin-3-yl)butyl)thiazole-5-carbaldehyde oxime 20:**

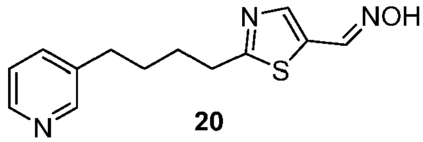

**20**

To a solution of (E/Z)-2-(4-(pyridin-3-yl)but-1-yn-1-yl)thiazole-5-carbaldehyde oxime **19** (40 mg, 0.0155 mmol) in 4:1 ratio of EtOAc/MeOH (5 mL) was added 10% Pd/C (40 mg) at room temperature under Argon atmosphere and stirred the mixture for 2 h under $H_2$ atmosphere using balloon pressure. Upon completion, the mixture was filtered through a small celite pad and concentrated under reduced pressure and the crude was purified by column chromatography (EtOAc, 100%) to afford cis/trans isomers (9.4/0.6 ratio) of (E/Z)-2-(4-(pyridin-3-yl)butyl)thiazole-5-carbaldehyde oxime **20** as white solid (35 mg, 86%); $R_f$ (100% EtOAc) 0.20; IR (neat): $v_{max}$ 2949, 1578, 1461, 1421, 1188, 1050, 1036, 924, 801, 708, 664, 643, 662 $cm^{-1}$; HRMS ($ESI^+$): *m/z* calcd for $C_{13}H_{16}N_3OS^+$ 260.100860 found 262.101595; $^1H$ NMR (400 MHz, $CD_3OD$): δ ppm 8.48-8.30 (m, 2H), 8.26 (s, 0.06H) 8.00 (s, 0.94H), 7.78 (s, 0.94H), 7.74 (s, 0.06H) 7.70 (d, *J* = 7.9 Hz, 1H), 7.36 (dd, *J* = 7.7, 4.9 Hz, 1H), 3.08 (t, *J* = 7.4 Hz, 2H), 2.72 (t, *J* = 7.6 Hz, 2H), 1.89-1.82 (m, 2H), 1.78-1.70 (m, 2H); $^{13}C$ NMR (101 MHz, $CD_3OD$): δ ppm 175.68, 148.63, 146.16, 144.88, 138.37, 137.61, 136.88, 125.52, 123.79, 31.91, 31.86, 30.09, 29.02.

**2-(4-(pyridin-3-yl)butyl)thiazole-4-carbaldehyde oxime hydrochloride NM-143:**

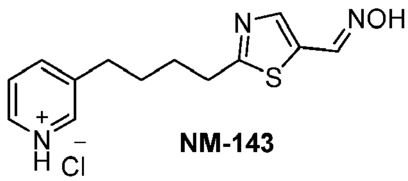

**NM-143**

To a solution of (E/Z)-2-(4-(pyridin-3-yl)butyl)thiazole-5-carbaldehyde oxime **20** (30 mg, 0.06 mmol) was added 2N HCl (2.5 mL) at room temperature and stirred for 30 min at same temperature. Upon completion, solvent was distilled off under reduced pressure and the resulting solid was washed with diethyl ether (2 x 5 mL). The solid was dried under

vacuum to give cis/trans isomers (8/2 ratio) of (E/Z)-2-(4-(pyridin-3-yl)butyl)thiazole-5-carbaldehyde oxime hydrochloride **NM-143** as a light brown solid (32 mg, 93.8%); IR (neat): $v_{max}$ 2934, 2360, 1555, 1468, 1219, 997, 930, 797, 734, 681 $cm^{-1}$; HRMS ($ESI^+$): *m/z* calcd for $C_{13}H_{16}N_3OS^+$ 262.100860 found 262.101253; $^1H$ NMR (400 MHz, $CD_3OD$): δ ppm 8.85 (s, 1H), 8.75 (d, *J* = 5.4 Hz, 1H), 8.61 (d, *J* = 7.7 Hz, 1H), 8.55 (s, 0.8H), 8.34 (s, 0.2H), 8.24 (s, 0.2H), 8.08-8.04 (m, 1H), 7.99 (s, 0.8H), 3.48-3.35 (m, 2H), 3.04-2.94 (m, 2H), 2.07-1.81 (m, 4H); $^{13}C$ NMR (101 MHz, $CD_3OD$): δ ppm 178.40, 178.08, 148.40, 144.05, 142.33, 140.48, 140.21, 136.80, 136.27, 136.23, 134.03, 128.50, 128.20, 32.82, 31.48, 30.80, 30.66, 30.62, 29.85, 29.79.

**5-(4-(quinolin-4-ylamino)but-1-yn-1-yl)furan-2-carbaldehyde oxime NM-152:**

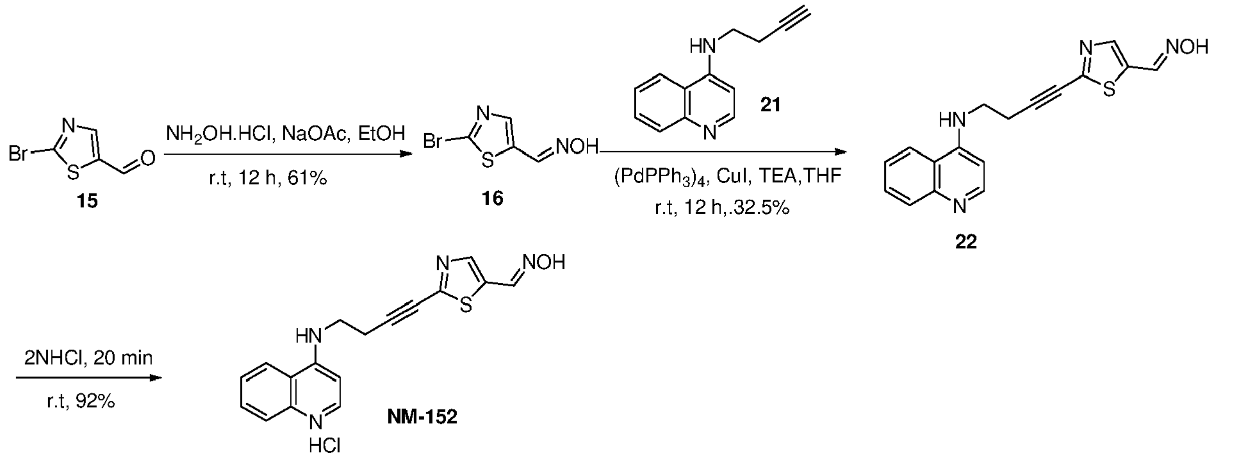


**2-(4-(quinolin-4-ylamino)but-1-ynyl)thiazole-5-carbaldehyde oxime 22:**

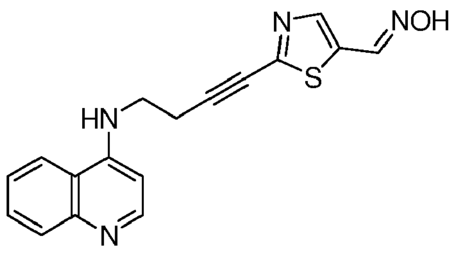

**22**

To a degassed solution of (E/Z)-2-bromothiazole-5-carbaldehyde oxime **16** (69 mg, 0.333 mmol, 1.1 equiv) in THF/$Et_3N$ (6 mL/ 2 mL), Pd[$PPh_3$]$_4$ (53 mg, 0.045 mmol, 0.15 equiv) and CuI (17 mg, 0.0892 mmol, 0.3 equiv) were added. After degassing the reaction mixture for 5 min at room temperature, N-(but-3-yn-1-yl)quinolin-4-amine **21** (60 mg, 0.305 mmol, 1.0 equiv) was added dropwise and the reaction mixture was stirred at the room temperature for 16 h. After completion, the reaction mixture was concentrated under reduced pressure and the residue was purified by column chromatography (MeOH/$CH_2Cl_2$, 10:90) to afford cis-trans isomers (94/6) of (E/Z)-2-(4-(quinolin-4-ylamino)but-1-ynyl)thiazole-5-carbaldehyde oxime **22** as pale yellow solid (32 mg, 32.5%); $R_f$ (10% MeOH/$CH_2Cl_2$) 0.20; HRMS ($ESI^+$): m/z calcd for $C_{17}H_{15}N_4OS^+$ 323.096109 found 323.095000; $^1H$ NMR (400 MHz, DMSO-de): δ ppm 12.46 (s, 0.94H), 11.64 (s, 0.06H), 9.14 (s, 1H), 8.57 (d, *J* = 6.8 Hz, 1H), 8.50 (d, *J* = 8.5 Hz, 1H), 8.38 (s, 0.06H), 8.20 (s, 0.94H), 8.01 (s, 0.94H), 7.98 (s, 0.06H), 7.96-7.86 (m, 2H), 7.71 (ddd, J= 8.3, 5.6, 2.6 Hz, 1H), 7.02 (d, *J* = 6.9 Hz, 0.94H), 6.79 (d, *J* = 6.8 Hz, 0.06H), 3.92-3.80 (m, 2H), 3.03 (t, *J* = 6.7 Hz, 2H); $^{13}C$ NMR (101 MHz, DMSO-de): δ ppm 155.29, 150.95, 147.05, 144.08, 139.63, 137.94, 133.43, 126.86, 126.28, 123.25, 121.91, 117.37, 99.01, 96.24, 75.87, 41.47, 19.55.

**2-(4-(quinolin-4-ylamino)but-1-ynyl)thiazole-5-carbaldehyde oxime hydrochloride NM-152:**

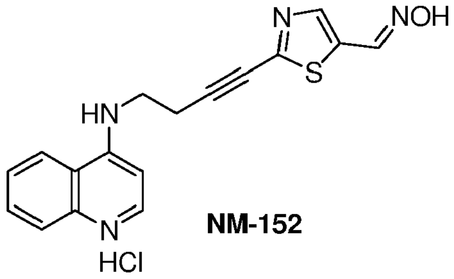

NM-152

To a solution of (E/Z)-2-(4-(quinolin-4-ylamino)but-1-ynyl)thiazole-5-carbaldehyde oxime 22 (20 mg, 0.062 mmol) was added 2N HCl (2 mL) at room temperature and stirred for 20 min at same temperature. Upon completion, solvent was distilled off under reduced pressure and the resulting solid was washed with diethyl ether (2 x 4 mL). The solid was dried under vacuum to give cis-trans mixture of (E/Z)-2-(4-(quinolin-4-ylamino)but-1-ynyl)thiazole-5-carbaldehyde oxime hydrochloride as a light brown solid **NM-152** (20.5 mg, 92%); HRMS (ESI$^+$): *m/z* calcd for $C_{17}H_{15}N_4OS^+$ 323.096109 found 323.0951581; $^1$H NMR (400 MHz, $CD_3OD$): δ ppm 8.59-8.31 (m, 2.5H), 8.26-8.05 (m, 0.5H), 8.03-7.81 (m, 2.5H), 7.80-7.59 (m, 1H), 7.44 (bs, 0.5H), 7.15-6.94 (m, 1H), 4.07 (s, 2H), 3.34 (d, 2H); $^{13}$C NMR (101 MHz, $CD_3OD$): δ ppm 156.39, 145.43, 144.72, 144.46, 142.32, 139.78, 137.88, 137.54, 136.18, 133.72, 133.58, 127.11, 126.85, 122.67, 122.33, 119.82, 119.68, 118.57, 117.07, 98.47, 98.43, 41.33, 41.29, 39.24, 39.19.

**2-(4-(quinolin-4-ylamino)but-1-ynyl)thiazole-4-carbaldehyde hydrochloride NM-176:**

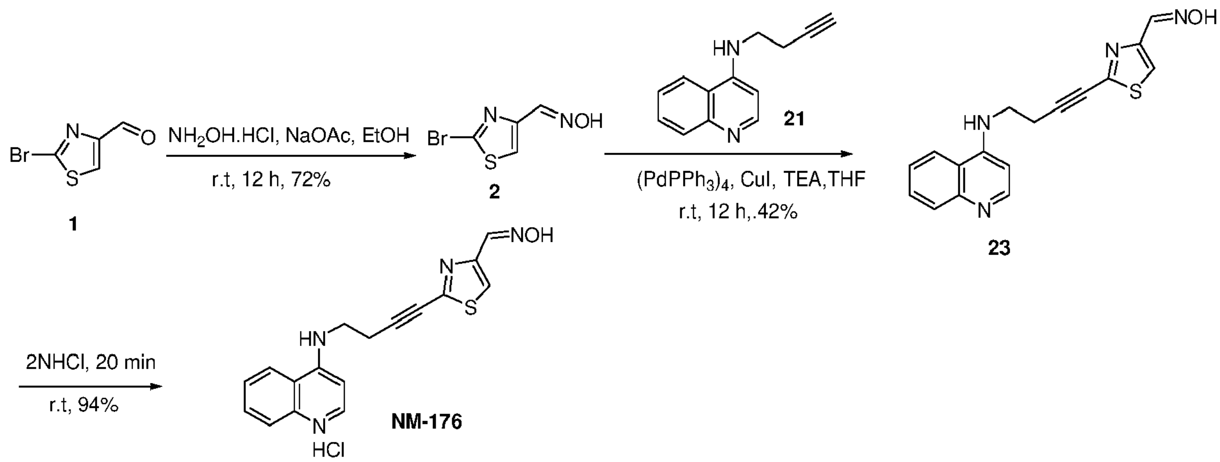


**2-(4-(quinolin-4-ylamino)but-1-ynyl)thiazole-4-carbaldehyde oxime 23:**

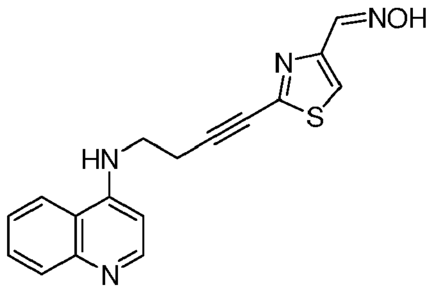

**23**

To a degassed solution of (E/Z)-2-bromothiazole-4-carbaldehyde oxime **2** (69 mg, 0.570 mmol, 1.1 equiv) in THF/$Et_3N$ (6 mL/ 2 mL), Pd[$PPh_3$]$_4$ (53 mg, 0.0778 mmol, 0.15 equiv) and CuI (17 mg, 0.155 mmol, 0.3 equiv) were added. After degassing the reaction mixture for 5 min at room temperature, N-(but-3-yn-1-yl)quinolin-4-amine **15** (100 mg, 0.519 mmol, 1.0 equiv) was added dropwise and the reaction mixture was stirred at the room temperature for 16 h. After completion, the reaction mixture was concentrated under reduced pressure and the residue was purified by column chromatography (MeOH/$CH_2Cl_2$, 10:90) to afford cis-trans isomers (7.5/2.5) of (E/Z)-2-(4-(quinolin-4-ylamino)but-1-ynyl)thiazole-5-carbaldehyde oxime **23** as a pale yellow solid (32 mg, 42%); $R_f$ (10% MeOH/$CH_2Cl_2$) 0.20; HRMS (ESI$^+$): *m*/*z* calcd for $C_{17}H_{15}N_4OS^+$ 323.096109 found 323.0957321; $^1$H NMR (400 MHz, DMSO-de): δ ppm 12.17 (s, 0.75H), 11.45 (s, 0.25H), 8.52 (s, 0.75H), 8.44 (s, 0.75H), 8.22 (d, *J* = 8.3 Hz, 1H), 8.15 (s, 0.25H), 7.92 (s, 0.25H), 7.81 (d, *J* = 8.0 Hz, 1H), 7.69-7.58 (m, 2H), 7.53-7.39 (m, 2H), 6.62 (d, *J* = 5.1 Hz, 1H), 3.63 (dd, *J* = 12.7, 6.6 Hz, 2H), 2.94 (t, *J* = 6.8 Hz, 2H); $^{13}$C NMR (101 MHz, DMSO-$d_6$): δ ppm 150.81, 150.15, 149.74, 148.60, 147.13, 145.68, 143.17, 139.55, 129.43, 129.23, 127.19, 124.55, 122.09, 120.39, 95.40, 75.19, 74.98, 41.15, 19.41, 19.37.

**2-(4-(quinolin-4-ylamino)but-1-ynyl)thiazole-4-carbaldehyde oxime hydrochloride NM-176:**

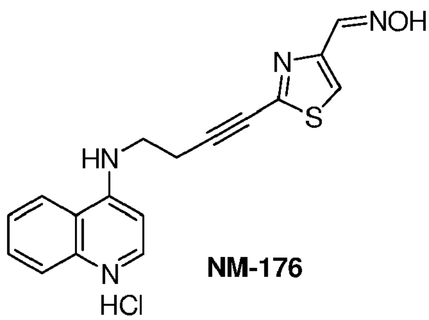

**NM-176**

To a solution of (E/Z)-2-(4-(quinolin-4-ylamino)but-1-ynyl)thiazole-4-carbaldehyde oxime **23** (20 mg, 0.062 mmol) was added 2N HCl (2 mL) at room temperature and stirred for 20 min at same temperature. Upon completion, solvent was distilled off under reduced pressure and the resulted solid was washed with diethyl ether (2 x 4 mL). The solid was dried under vacuum to give cis-trans mixture of (E/Z)-2-(4-(quinolin-4-ylamino)but-1-ynyl)thiazole-4-carbaldehyde oxime hydrochloride **NM-176** as a light brown solid (21 mg, 94%); HRMS (ESI$^+$): *m*/*z* calcd for $C_{17}H_{15}N_4OS^+$ 323.096109 found 323.0959331; $^1$H NMR (400 MHz, $CD_3OD$): δ ppm 8.53-8.37 (m, 2.25H), 8.18-7.80 (m, 2.75H), 7.73 (dd, *J* = 17.0, 8.5 Hz, 1H), 7.64-7.49 (m, 0.5H), 7.45-7.22 (m, 0.5H), 7.12-6.95 (m, 1H), 4.09-3.90 (m, 2H), 3.23 (dt, *J* = 16.4, 6.5 Hz, 1H), 3.13-3.00 (m, 1H); $^{13}$C NMR (101 MHz, $CD_3OD$): δ ppm 156.59, 156.43, 142.15, 142.04, 141.98, 141.98, 141.94, 137.92, 137.88, 133.67, 133.67, 127.05, 127.01, 122.53, 122.46, 119.78, 117.07, 117.00, 98.33, 98.30, 41.28, 41.14, 19.12, 18.99.

**4-(2-(pyridin-3-yl)ethyl)thiazole-2-carbaldehyde oxime hydrochloride NM-171:**

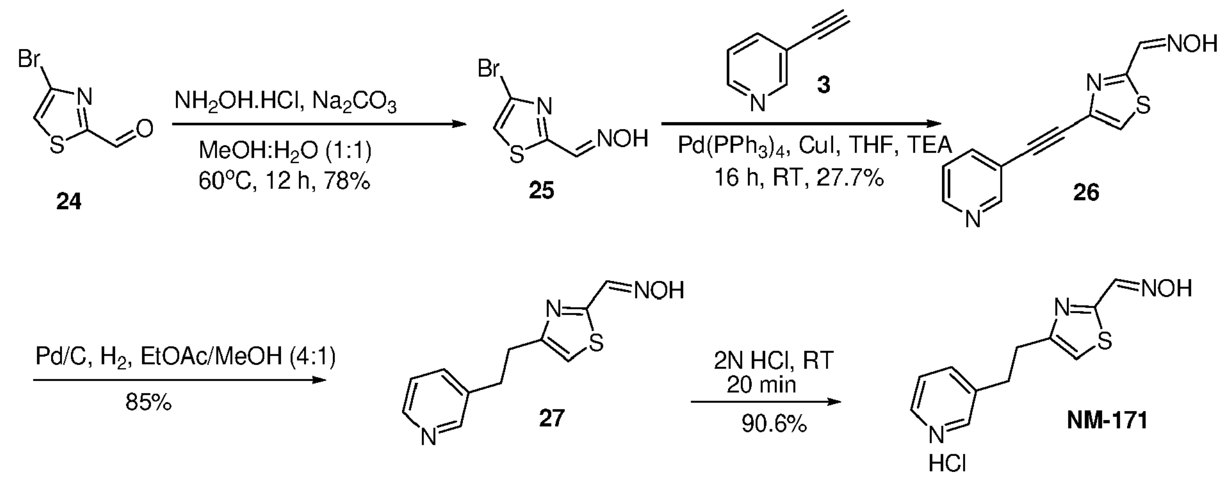


24 25 26 27 NM-171

**4-bromothiazole-2-carbaldehyde oxime 25:**

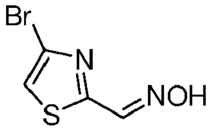

**25**

A solution of commercially available 4-bromothiazole-2-carbaldehyde **24** (1 g, 5.20 mmol, 1 equiv), hydroxylamine hydrochloride (723 mg, 10.40 mmol, 2 equiv) and $Na_2CO_3$ (1.655 g, 15.61 mmol, 3 equiv) in 1:1 ratio of MeOH/$H_2O$ (20 mL) was stirred at 60 °C for 2 h. Upon completion, the reaction mixture was concentrated under reduced pressure and 30 mL of water was added. The mixture was extracted with EtOAc (2x 30 mL), dried over anhydrous $Na_2SO_4$, concentrated under reduced pressure and the residue was purified by column chromatography (EtOAc/PE 2:8) to afford cis-trans isomers (1/0.66) of (E/Z)-4-bromothiazole-2-carbaldehyde oxime **25** as an off white solid (824 mg, 78%); $R_f$ (20% EtOAc/PE) 0.35; $^1$H NMR (400 MHz, Acetone-de): δ ppm 12.19 (s, 1H), 11.28 (s, 0.66H), 8.29 (d, $J$ = 0.8 Hz, 0.66H), 7.93 (d, $J$ = 0.8 Hz, 1H), 7.90 (d, $J$ = 1.0 Hz, 1H), 7.65 (d, $J$ = 0.8 Hz, 0.66H); $^{13}$C NMR (101 MHz, Acetone-de): δ ppm 143.50, 139.97, 126.00, 125.10, 122.16, 118.46.

**4-(pyridin-3-ylethynyl)thiazole-2-carbaldehyde oxime 26:**

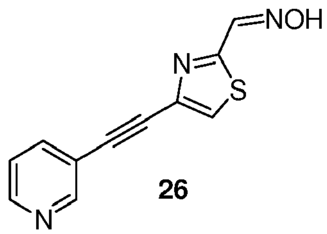

**26**

To a degassed solution of (E/Z)-4-bromothiazole-2-carbaldehyde oxime **25** (165.8 mg, 0.80 mmol, 1.1 equiv) in THF/$Et_3N$ (9 mL/ 3 mL), Pd[$PPh_3$]$_4$ (126.3 mg, 0.109 mmol, 0.15 equiv) and CuI (41.6 mg, 0.109 mmol, 0.3 equiv) were added. After degassing the reaction mixture for 5 min at room temperature, 3-ethynylpyridine **3** (75 mg, 0.728 mmol, 1 equiv) was added dropwise and the reaction mixture was stirred at the room temperature for 16 h. Upon completion, the reaction mixture was concentrated under reduced pressure and the residue was purified by column chromatography (EtOAc/PE 50:50) to give cis-trans isomers (7/3, ratio) of (E/Z)-4-(pyridin-3-ylethynyl)thiazole-2-carbaldehyde oxime **26** as an off white solid (46 mg, 27.7%); $R_f$ (50% EtOAc/PE) 0.25; HRMS (ESI$^+$): $m/z$ calcd for $C_{11}H_8N_3OS^+$

230.038259 found 230.039853; $^1$H NMR (400 MHz, Acetone-de): δ ppm 8.79 (s, 1H), 8.64 (dd, *J* = 9.3, 8.0 Hz, 1H), 8.33 (d, *J* = 0.8 Hz, 0.7H), 8.20 (d, *J* = 0.9 Hz, 0.3H), 8.03-7.92 (m, 2H), 7.47 (dd, *J* = 7.5, 5.2 Hz, 1H); $^{13}$C NMR (101 MHz, Acetone-de): δ ppm 162.24, 151.94, 149.33, 143.92, 143.83, 138.42, 137.26, 128.16, 124.98, 123.41, 119.30, 86.13, 85.35.

**2-(2-(pyridin-3-yl)ethyl)thiazole-4-carbaldehyde oxime 27:**

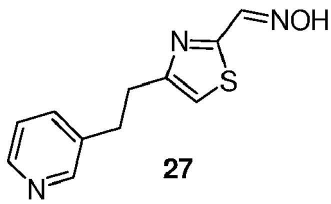

**27**

To a solution of (E/Z)-4-(pyridin-3-ylethynyl)thiazole-2-carbaldehyde oxime **26** (20 mg, 0.087 mmol) in EtOAc (3 mL) was added 10% Pd/C (10 mg) at room temperature under Argon atmosphere and stirred the mixture for 1 h under $H_2$ atmosphere using balloon pressure. Upon completion, the mixture was filtered through a small celite pad, concentrated under reduced pressure followed by column chromatography (10% MeOH/$CH_2Cl_2$) to afford (E/Z)-4-(2-(pyridin-3-yl)ethyl)thiazole-4-carbaldehyde oxime **27** as an off white solid (17.3 mg, 85%). $R_f$ (100% EtOAc) 0.20.

**4-(2-(pyridin-3-yl)ethyl)thiazole-2-carbaldehyde oxime hydrochloride NM-171:**

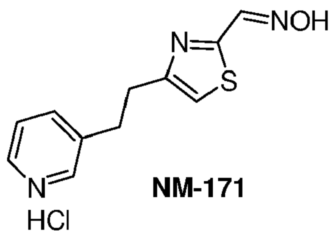

**NM-171**

To a solution of (E/Z)-4-(2-(pyridin-3-yl)ethyl)thiazole-2-carbaldehyde oxime **27** (11 mg, 0.085 mmol) in $CH_2Cl_2$ (3 ml) was added 2N HCl (1 mL) at room temperature and stirred for 30 min at same temperature. Upon completion, solvent was distilled off under reduced pressure and the resulting solid was washed with diethyl ether (2 x 3 mL). The solid was dried under vacuum to give mixture of cis-trans isomers (1/1, ratio) of (E/Z)-4-(2-(pyridin-3-yl)ethyl)thiazole-2-carbaldehyde oxime hydrochloride **NM-171** as a light brown solid (11.5 mg, 90.6%); HRMS (ESI$^+$): *m*/*z* calcd for $C_{11}H_{12}N_3OS^+$ 234.0696 found 234.0694; $^1$H NMR (400 MHz, $CD_3OD$): δ ppm 8.93-8.69 (m, 2H), 8.61 (d, *J* = 7.0 Hz, 1H), 8.14 (s, 0.5H), 8.08 (bs, 1H), 7.83 (s, 0.5H), 7.70-7.55 (m,1H), 3.55-3.33 (m, 4H); $^{13}$C NMR (101 MHz, $CD_3OD$): δ ppm149.31, 147.19, 141.38, 141.27, 141.27, 141.14, 139.57, 138.88, 135.87, 132.44, 132.42, 131.73, 131.63, 128.67, 128.55, 127.21, 127.15, 121.58, 31.17, 31.14, 29.66, 29.08.

**2-(2-(pyridin-3-yl)ethyl)oxazole-5-carbaldehyde oxime NM-168:**

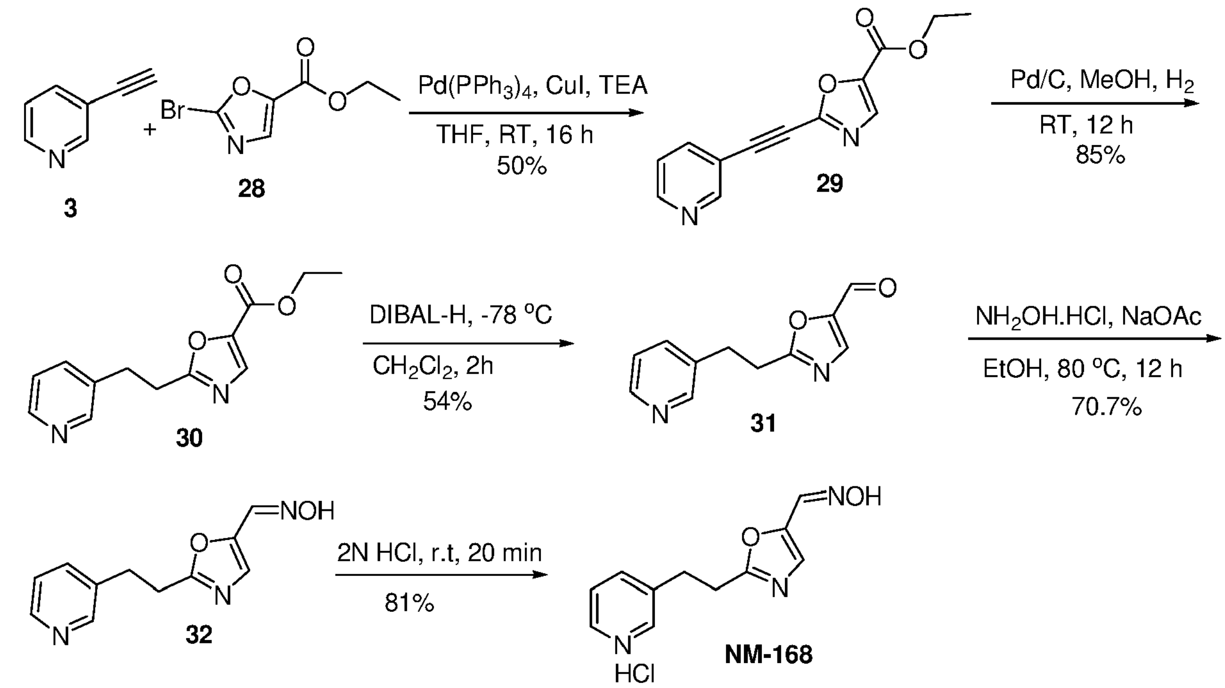

3 28 29 30 31 32 NM-168

**Ethyl 2-(pyridin-3-ylethynyl)oxazole-5-carboxylate 29:**

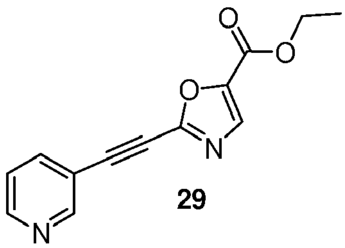
**29**

To a degassed solution of ethyl 2-bromooxazole-5-carboxylate **28** (470 mg, 2.135 mmol, 1.1 equiv) in THF/$Et_3N$ (12 mL/ 4 mL), $Pd[PPh_3]_4$ (336 mg, 0.290 mmol, 0.15 equiv) and CuI (110 mg, 0.577 mmol, 0.3 equiv) were added. After degassing the reaction mixture for 5 min at room temperature, 3-ethynylpyridine **3** (200 mg, 1.941 mmol, 1 equiv) was added dropwise and the reaction mixture was stirred at the room temperature for 16 h. Upon completion, the reaction mixture was concentrated under reduced pressure and the residue was purified by column chromatography (EtOAc/PE, 1:1) to afford ethyl 2-(2-(pyridin-3-yl)ethyl)oxazole-5-carboxylate **29** as an off white solid (220 mg, 46.8%); HRMS (ESI$^+$): *m/z* calcd for $C_{13}H_{10}N_2NaO_3^+$ 265.058363 found 265.059171; $R_f$ (50% EtOAc/PE) 0.35; $^1$H NMR (400 MHz, $CDCl_3$): δ ppm 8.85 (bs, 1H), 8.68 (bs, 1H), 8.28 (s, 1H), 7.90 (d, *J* = 7.9 Hz, 1H), 7.37 (dd, *J* = 7.5, 5.0 Hz, 1H), 4.43 (q, *J* = 7.1 Hz, 2H), 1.42 (t, *J* = 7.1 Hz, 3H); $^{13}$C NMR (101 MHz, $CDCl_3$): δ ppm 160.38, 152.74, 150.42, 146.63, 144.53, 139.17, 134.71, 89.04, 79.17, 77.34, 77.02, 76.71, 61.58, 14.26.

**Ethyl 2-(2-(pyridin-3-yl)ethyl)oxazole-5-carboxylate 30:**

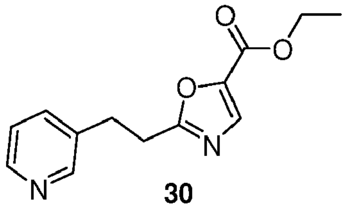
**30**

To a solution of ethyl 2-(2-(pyridin-3-yl)ethyl)oxazole-5-carboxylate **29** (120 mg, 0.495 mmol) in MeOH (10 mL) was added 10% Pd/C (60 mg) at room temperature under Argon atmosphere and stirred the mixture for 12 h under $H_2$ atmosphere using balloon pressure. Upon completion, the mixture was filtered through a small celite pad, concentrated under reduced pressure and purified by $SiO_2$ column chromatography (EtOAc/PE, 70:30) ethyl 2-(2-(pyridin-3-yl)ethyl)oxazole-5-carboxylate **30** as a light brown syrup (91 mg, 74.6%); $R_f$ (50% EtOAc+PE) 0.3; HRMS (ESI+): *m/z* calcd for $C_{13}H_{14}N_2NaO_3^+$ 269.089663 found 269.089903; $^1$H NMR (400 MHz, $CDCl_3$): δ ppm 8.42 (bs, 2H), 8.12 (s, 1H), 7.50 (dd, *J* = 6.2, 1.6 Hz, 1H), 7.21 (dd, *J* = 7.7, 4.8 Hz, 1H), 4.36 (q, *J* = 7.1 Hz, 2H), 3.12 (bs, 4H), 1.36 (t, *J* = 7.1 Hz, 3H); $^{13}$C NMR (101 MHz, $CDCl_3$): δ ppm 164.16, 161.16, 149.72, 148.08, 143.73, 135.70, 135.12, 133.52, 123.49, 61.18, 30.09, 29.48, 14.28.

**2-(2-(pyridin-3-yl)ethyl)oxazole-5-carbaldehyde oxime 32:**

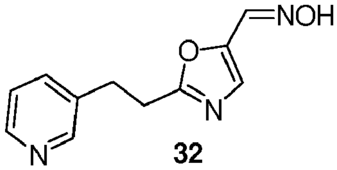

**32**

To a solution of ethyl 2-(2-(pyridin-3-yl)ethyl)oxazole-5-carboxylate **30** (80 mg, 0324 mmol, 1 equiv) in $CH_2Cl_2$ (4 mL) was added DIBAL-H (0.8 mL, 1M in THF, 0.809 mmol, 2.5 equiv) at -78 °C and stirred over 2 h at same temperature. The mixture was quenched with MeOH (2 mL) at -78 °C and concentrated under reduced pressure. The white aluminum salts were removed by filtered in $CH_2Cl_2$, concentrated under reduced pressure and purified by column chromatography to give 2-(2-(pyridin-3-yl)ethyl)oxazole-5-carbaldehyde **31** as an off white solid (22 mg, 53.6% w.r.t SM, 30 mg SM recovered). $^1$H NMR (400 MHz, $CDCl_3$): δ 9.92 (s, 1H), 8.50 (d, *J* = 2.3 Hz, 2H), 8.19 (d, *J* = 4.4 Hz, 1H), 7.57-7.53 (m, 1H), 7.25 (dd, J = 7.8, 4.2 Hz, 1H), 3.18 (s, 4H). To a solution of 2-(2-(pyridin-3-yl)ethyl)oxazole-5-carbaldehyde **31** (20 mg, 0.0989 mmol, 1 equiv) in EtOH (5 mL) was added hydroxylamine hydrochloride (13.7 mg, 0.1971 mmol, 2 equiv), NaOAc (24.34 mg, 2.429 mmol, 3 equiv) and refluxed for 16 h. After concentration under reduced pressure, the crude product was purified by $SiO_2$ column chromatography (100% EtOAc) to afford cis-trans isomers of (8/2, ratio) of (E/Z)-2-(2-(pyridin-3-yl)ethyl)oxazole-5-carbaldehyde oxime **32** as an off white solid (15.2 mg, 70.7%); $R_f$ (100% EtOAc) 0.35; HRMS (ESI+): *m/z* calcd for $C_{11}H_{12}N_3O_2^+$ 218.092403 found 218.091853; $^1$H NMR (400 MHz, $CDCl_3$): δ ppm 8.51 (bs, 2H), 8.43 (s, 0.8H), 8.06 (s, 0.2H), 7.77 (s, 0.2H), 7.61-7.50 (m, 1.8H), 7.28-7.23 (m, 1H), 3.17 (bs, 4H); $^{13}$C NMR (101 MHz, $CDCl_3$): δ ppm 162.59, 149.53, 147.74, 143.02, 136.16, 135.52, 123.64, 30.07, 29.28.

**2-(2-(pyridin-3-yl)ethyl)oxazole-5-carbaldehyde oxime hydrohloride NM-168:**

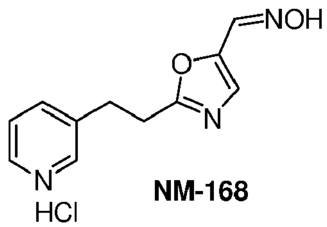

**NM-168**

To a solution of ((E/Z)-2-(2-(pyridin-3-yl)ethyl)oxazole-5-carbaldehyde oxime **32** (13.5 mg, 0.062 mmol) in $CH_2Cl_2$ (2 ml) was added 2N HCl (2 mL) at room temperature and stirred for 20 min at same temperature. Upon completion, solvent was distilled off under reduced pressure and the resulting solid was washed with diethyl ether (2 x 3 mL). The solid was dried under vacuum to give (E)-2-(2-(pyridin-3-yl)ethyl)oxazole-5-carbaldehyde oxime hydrochloride **NM-168** as an off white solid (12.8 mg, 81.3%); $^1$H NMR (400 MHz, $CD_3OD$): δ ppm 8.88 (s, 1H), 8.77 (d, *J* = 5.7 Hz, 1H), 8.63 (d, *J* = 8.1 Hz, 1H), 8.52 (s, 1H), 8.07 (dd, *J* = 8.0, 5.8 Hz, 1H), 7.37 (s, 1H), 3.42 (t, *J* = 7.0 Hz, 2H), 3.32-3.27 (m, 2H); $^{13}$C NMR (101 MHz, $CD_3OD$): δ ppm 162.42, 147.09, 143.08, 141.33, 141.23, 141.16, 139.44, 137.95, 127.03, 28.69, 27.33.

Example 2: ***in vitro* reactivation of human acetylcholinesterase (hAChE) by compounds of the invention**

Compounds NM-55, NM-109, NM-130, NM-131, NM-132, NM-139, NM-141, NM-143, NM-152, NM-168, NM-171 and NM-176 of the invention were tested for their reactivation properties of hAChE inhibited by O-ethyl S-[2-(diisopropylamino)ethyl] methylphosphonothioate (VX), tabun, sarin or paraoxon.
2-PAM (pralidoxime or 2-[(E)-(hydroxyimino)methyl]-1-methylpyridinium) and HI6 (asoxime chloride or [1-[(4-carbamoylpyridin-1-ium-1-yl)methoxymethyl]pyridin-2-ylidene]methyl-oxoazanium dichloride) were used as comparative compounds.

**Inhibition of hAChE by OPNAs.** Recombinant hAChE was produced and purified as previously described (see reference https://www.ncbi.nlm.nih.gov/pubmed/31132435). VX, sarin and tabun have been supplied by DGA maîtrise NRBC (Vert le Petit, France).
Stock solutions of OPNA at 5 mM in isopropanol were used to inhibit the purified *h*AChE as previously described [Carletti, E. et al. 2008]. Briefly, a ten-fold excess of OPNA was used to perform the inhibition of *h*AChE in a sodium phosphate buffer (100 mM, pH 7.4, 0.1% BSA) at 25°C. Complete inhibition of *h*AChE was monitored by measuring the residual activity with a modified Ellman assay as previously described [Ellman, G.L., et al. 1961] and excess of OPNA were removed by using a desalting PD-10 column (GE Healthcare).

**$IC_{50}$ measurements.** Compounds were dissolved in water to make 40 mM stock solutions. Recombinant *h*AChE activity was measured spectrophotometrically at 25°C, monitoring the absorbance at 412 nm, in 1 mL of Ellman's buffer (0.5 mM DTNB, 0.1% BSA, 0.1 M phosphate, pH 7.4), in the presence of appropriate oxime concentrations. Measurements were performed at least in duplicate for each concentration tested. The oxime concentration producing 50% inhibition was determined by nonlinear fitting with ProFit (Quantumsoft) using the standard IC 50 equation: % activity = 100×IC50/(IC50+ [Ox]).

**Reactivation of hAChE inhibited by OPNAs.** The ability of the compounds to reactivate OP-inhibited hAChE were assessed with a modified Ellman assay using a microplate reader (SPARK 10M, Tecan) and a continuous method described previously [Kitz, R.J., et al. 1965 , Worek, F., et al., 2004] with minor modifications. Briefly, the desired oximes concentrations to be tested were dispensed in a 96-well flat-bottomed polystyrene microplate containing 0.1% BSA phosphate buffer and DTNB. At t=0, OP-inhibited hAChE and acetylthiocholine (ATCh) diluted in 0.1% BSA phosphate buffer were injected in each well containing oximes using the built-in injectors of the microplate reader to a final volume of 200 μL. ATCh hydrolysis was continuously monitored over 30 minutes and the increase of absorbance at 412 nm recorded every 10 seconds at 25°C. Activities were individually corrected for oxime-induced hydrolysis of ATCh.
Reactivation of OP-inhibited hAChE by oximes proceeds according to scheme 1 and kinetics of oximes reactivation were determined as previously described [Worek, F., et al., 2004]. For each oxime concentration, the apparent reactivation rate, $k_{obs}$, the dissociation constant, $K_D$ and the reactivation rate constant, $k_r$, were calculated by nonlinear fitting with ProFit (Quantumsoft) using the standard oxime-concentration-dependent reactivation equation (1):

Scheme 1

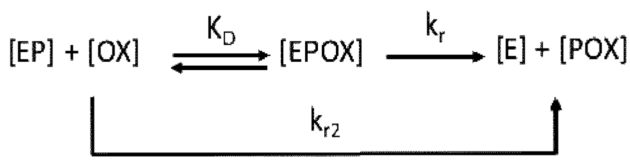


Eq (1): $$k_{obs} = \frac{k_r[OX]}{K_D + [OX]}$$

When [OX] << $K_D$, Eq (1) simplifies to Eq (2):

Eq (2) : $$k_{obs} = \left(\frac{k_r}{K_D}\right)[OX]$$

The second order reactivation rate constant $k_{r2}$, describing the specific reactivity can be derived from Eq (2).

Eq (3): $$k_{r2} = \frac{k_r}{K_D}$$

For the continuous method of recording OP-inhibited hAChE reactivation by oximes, the velocity of substrate hydrolysis (*v*) is proportional to the concentration of the reactivated hAChE and is expressed and derived as equation 4 and 5 respectively. $v_t$ is the velocity at time t and $v_0$ represents the maximum velocity. Equation 5 was used to determine the $k_{obs}$ by non-linear regression analysis for each individual oxime concentration with ProFit (Quantumsoft).

Eq (4) : $$v_t = v_0\,(1 - e^{-k_{obs}\,t})$$

Eq (5) : $$-d[S] = \int_0^t v dt = v_0 t + \frac{v_0}{k_{obs}}\,(e^{-k_{obs}t} - 1)$$

The results are as follows:

Table 1: Reactivation of OP-inhibited human hAChE by oximes 2-PAM, HI-6 and NMs

| OP | Oximes | $k_r$ ($min^{-1}$) | $K_D$ (µM) | $k_{r2}$ ($mM^{-1}.min^{-1}$) |
|---|---|---|---|---|
| VX | 2-PAM | 0.2 ± 0.01 | 26 ± 7 | 7 |
| | HI-6 | 0.4 ± 0.02 | 19 ± 4 | 20 |
| | NM-55 | 0.06 ± 0.002 | 6 ± 1 | 10 |
| | NM-109 | 0 | 0 | 0 |
| | NM-130 | 0 | 0 | 0 |
| | NM-131 | 0.1 ± 0.004 | 7 ± 1 | 14 |
| | NM-132 | 0.07 ± 0.002 | 2 ± 0,3 | 35 |
| Sarin | 2-PAM | 0.3 ± 0.02 | 25 ± 7 | 11 |
| | HI-6 | 0.8 ± 0.06 | 57 ± 11 | 13 |
| | NM-55 | 0.08 ± 0.003 | 3 ± 1 | 27 |
| | NM-109 | 0 | 0 | 0 |
| | NM-130 | 0 | 0 | 0 |
| | NM-131 | 0.08 ± 0.003 | 5 ± 1 | 16 |
| | NM-132 | 0.3 ± 0.002 | 3 ± 1 | 88 |
| Tabun | 2-PAM | 0.5 ± 0.2 | 211 ± 113 | 2 |
| | HI-6 | 0 | 0 | 0 |
| | NM-55 | 0 | 0 | 0 |
| | NM-109 | 0 | 0 | 0 |
| | NM-130 | 0 | 0 | 0 |
| | NM-131 | 0 | 0 | 0 |
| | NM-132 | 0 | 0 | 0 |
| Paraoxon | 2-PAM | 0.07 ± 0.02 | 68 ± 16 | 1 |
| | HI-6 | 0.8 ± 0.06 | 290 ± 70 | 0.4 |
| | NM-55 | 0.11 :!: 0.004 | 0.12 ± 0.03 | 917 |
| | NM-109 | 0 | 0 | 0 |
| | NM-130 | 0 | 0 | 0 |
| | NM-131 | 0.2 ± 0,006 | 12 ± 2 | 17 |
| | NM-132 | 0.3 ± 0.02 | 56 ± 11 | 5 |

Table 2: Reactivation of OP-inhibited human hAChE by oximes 2-PAM, HI-6 and NMs

| OP | Oximes | $k_r$ ($min^{-1}$) | $K_D$ ($\mu M$) | $k_{r2}$ ($mM^{-1}.min^{-1}$) |
|---|---|---|---|---|
| VX | 2-PAM | 0.2 ± 0.01 | 26 ± 7 | 7 |
| | HI-6 | 0.4 ± 0.02 | 19 ± 4 | 20 |
| | NM-139 | 0.2 ± 0,008 | 19 ± 4 | 8 |
| | NM-141 | 0.09 ± 0.004 | 1 ± 0.2 | 90 |
| | NM-143 | 0.07 ± 0.0007 | 0.1 ± 0.02 | 700 |
| | NM-152 | 0 | 0 | 0 |
| Sarin | 2-PAM | 0.3 ± 0.02 | 25 ± 7 | 11 |
| | HI-6 | 0.8 ± 0.06 | 57 ± 11 | 13 |
| | NM-139 | 0,18 ± 0,02 | 111 ± 22 | 1.6 |
| | NM-141 | 0.11 ± 0.002 | 45 ± 3 | 2.5 |
| | NM-143 | 0.1 ± 0.002 | 10 ± 1 | 10 |
| | NM-152 | 0 | 0 | 0 |
| Tabun | 2-PAM | 0.5 ± 0.2 | 211 ± 113 | 2 |
| | HI-6 | 0 | 0 | 0 |
| | NM-139 | 0 | 0 | 0 |
| | NM-141 | 0 | 0 | 0 |
| | NM-143 | 0 | 0 | 0 |
| | NM-152 | 0 | 0 | 0 |
| Paraoxon | 2-PAM | 0.07 ± 0.02 | 68 ± 16 | 1 |
| | HI-6 | 0.8 ± 0.06 | 290 ± 70 | 0.4 |
| | NM-139 | 0.6 ± 0.005 | 257 ± 45 | 2 |
| | NM-141 | 0.14 ± 0.005 | 6 ± 1 | 24 |
| | NM-143 | 0.3 ± 0.008 | 11 ± 2 | 27 |
| | NM-152 | 0 | 0 | 0 |

Table 3: IC50 for AChE of oximes: 2-PAM, HI6 and NMs

| Oxime | IC50 ($\mu M$) |
|---|---|
| 2-PAM | 580 ± 28 |
| HI-6 | 82 ± 6 |
| NM-55 | 57 ± 12 |
| NM-109 | 246 ± 28 |
| NM-130 | 590 ± 47 |
| NM-131 | 99 ± 12 |
| NM-132 | 133 ± 12 |
| NM-139 | 328 ± 30 |
| NM-141 | 114 ± 6 |
| NM-143 | 436 ± 22 |
| NM-152 | 1 ± 0,06 |
| NM-168 | 394 ± 33 |
| NM-171 | 398 ± 17 |
| NM-176 | 2 ± 0,01 |

These results show that the compounds of the invention have a broad spectrum of reactivation of OPNA-inhibited AChE: particularly they show an increased efficacy for VX and paraoxon, and a good potency against sarin.

**Claims**

1. Compound which is chosen from compounds of formula (I) and their pharmaceutically acceptable salts:

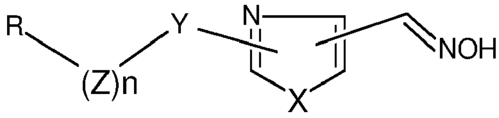

(I)

wherein:

X is O or S;
Y is $-CH_2-CH_2-$, $-C\equiv C-$ or -CH=CH-;
Z is $-CH_2-$,
n is an integer from 0 to 4; and
R is an alkyl group, an aryl, a heteroaryl, a cycloalkyl, a heterocyclyl, a biomolecule, a fluorescent probe, or a group -N(R1)(R2), wherein R1 and R2 are each independently H,
an alkyl group, an aryl, a heteroaryl or a cycloalkyl.

2. Compound according to claim 1, which is a salt of a compound of formula (I) with an acid or a base, preferably a chlorhydrate salt.

3. Compound according to claim 1 or 2, wherein R is a heteroaryl or a group -N(R1)(R2), wherein R1 and R2 are each independently H or a heteroaryl.

4. Compound according to any one of claims 1 to 3, wherein the heteroaryl group is a pyridine group such as 2-, 3- or 4-pyridino, or a quinoline group such as a 4-quinolinyl.

5. Compound according to any one of claims 1 to 4, wherein the -Y-(Z)n-R group or the oxime group =NOH is in position 2.

6. Compound according to any one of claims 1 to 5, wherein it is chosen from:

- compounds of formula (II) and their pharmaceutically acceptable salts:

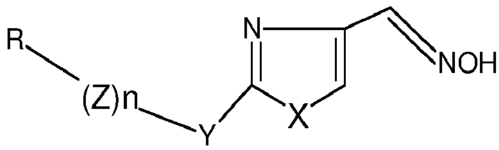

(II),

wherein X, Y, Z, n and R1 are as in claim 1 ;
- compounds of formula (III) and their pharmaceutically acceptable salts:

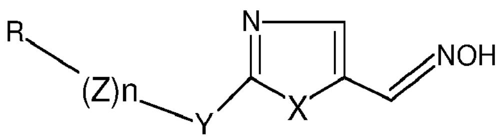

(III)

wherein X, Y, Z, n and R1 are as in claim 1 ;
- compounds of formula (IV) and their pharmaceutically acceptable salts:

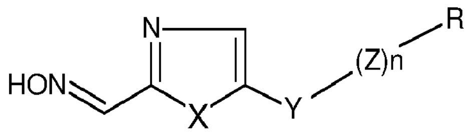

(IV)

wherein X, Y, Z, n and R1 are as in claim 1 ; and
- compounds of formula (V) and their pharmaceutically acceptable salts:

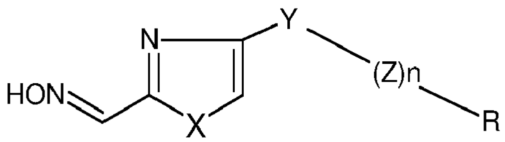

(V)

wherein X, Y, Z, n and R1 are as in claim 1.

**7.** Compound according to claim 6, wherein it is chosen from:

- compounds of formula (II) and their pharmaceutically acceptable salts, wherein :

X is S;
Y is $-CH_2-CH_2-$ or -C≡C-,
n is 0, 1 or 2, preferably n is 0 or 2; and
R is a heteroaryl or a group -N(R1)(R2), preferably said heteroaryl is not substituted and is a pyridine group such as 2-, 3- or 4-pyridino; or R1 is H and R2 is a heteroaryl, preferably not substituted, preferably a quinoline group such as a 4-quinolinyl; or
X is O;
Y is $-CH_2-CH_2-$ or -C≡C-,
n is 0, 1 or 2, preferably n is 0 or 2; and
R is a heteroaryl, preferably said heteroaryl is not substituted and is a pyridine group such as 2-, 3- or 4-pyridino;

- compounds of formula (III) and their pharmaceutically acceptable salts, wherein :

X is S;
Y is $-CH_2-CH_2-$ or -C≡C-,
n is 0, 1 or 2, preferably n is 0 or 2; and
R is a heteroaryl or a group -N(R1)(R2), preferably said heteroaryl is not substituted and is a pyridine group such as 2-, 3- or 4-pyridino; or R1 is H and R2 is a heteroaryl, preferably not substituted, preferably a quinoline group such as a 4-quinolinyl; or
X is O;
Y is $-CH_2-CH_2-$ or -C≡C-,
n is 0, 1 or 2, preferably n is 0 or 2; and
R is a heteroaryl, preferably said heteroaryl is not substituted and is a pyridine group such as 2-, 3- or 4-pyridino;

- compounds of formula (IV) and their pharmaceutically acceptable salts, wherein :

X is S;
Y is $-CH_2-CH_2-$ or -C≡C-,
n is 0, 1 or 2, preferably n is 0 or 2; and
R is a heteroaryl, preferably said heteroaryl is not substituted and is a pyridine group such as 2-, 3- or 4-pyridino; and

- compounds of formula (V) and their pharmaceutically acceptable salts, wherein :

X is S;

Y is $-CH_2-CH_2-$ or -C≡C-,

- n is 0, 1 or 2, preferably n is 0 or 2; and
R is a heteroaryl, preferably said heteroaryl is not substituted and is a pyridine group such as 2-, 3- or 4-pyridino.

**8.** Compound according to any one of claims 1 to 7, wherein it is chosen from:

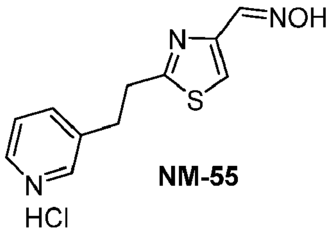

**NM-55**

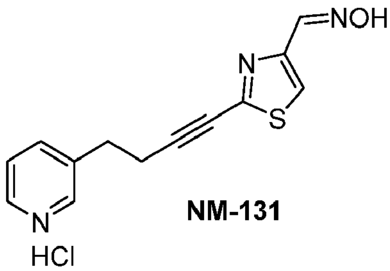

**NM-131**

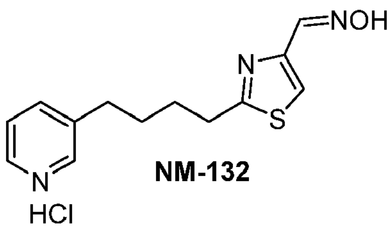

**NM-132**

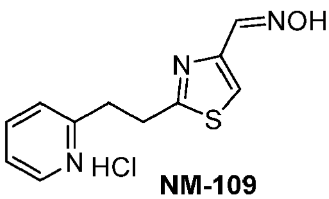

**NM-109**

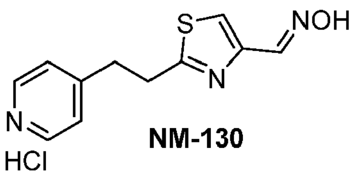

**NM-130**

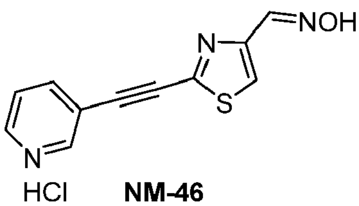

**NM-46**

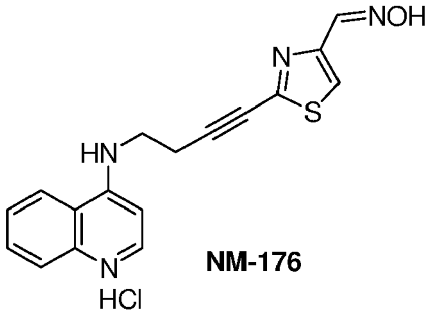

**NM-176**

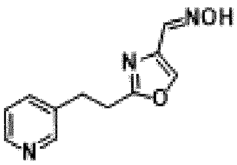

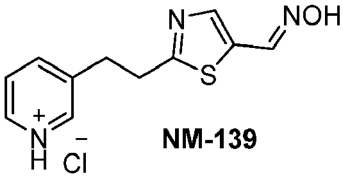

**NM-139**

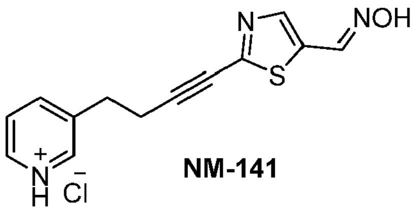

**NM-141**

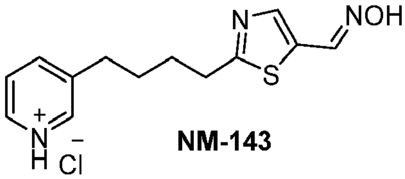

**NM-143**

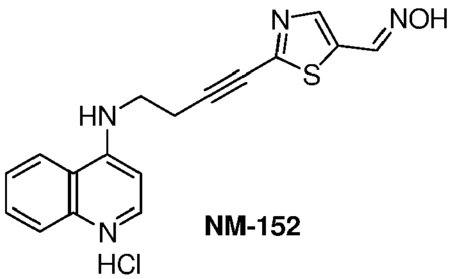

**NM-152**

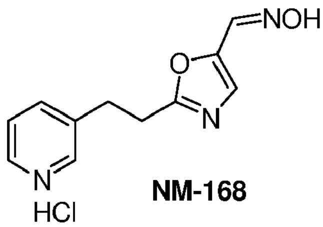

NM-168

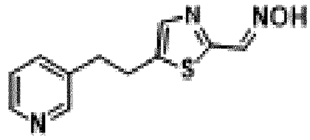

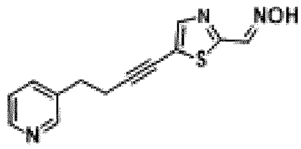

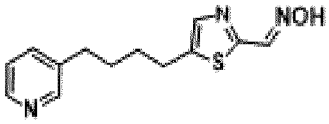

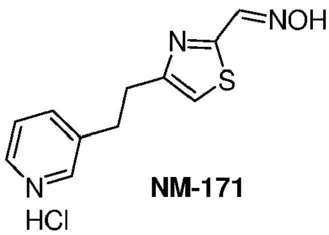

NM-171

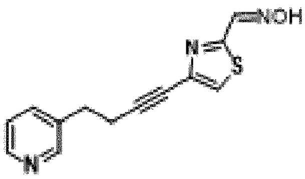

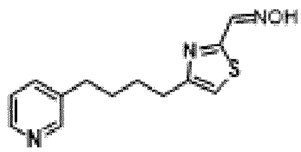

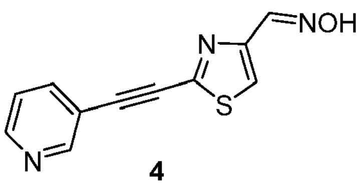

4

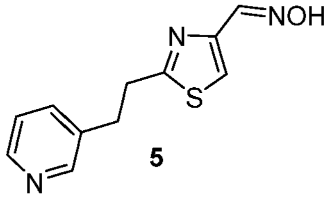

**5**

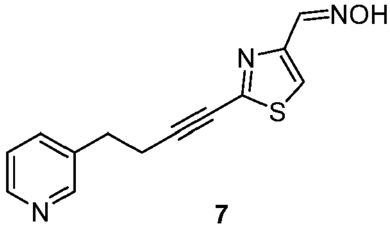

**7**

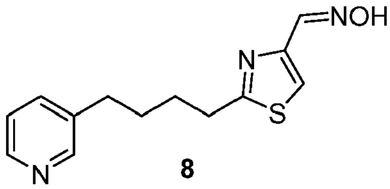

**8**

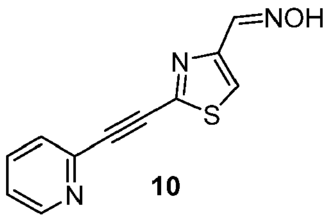

**10**

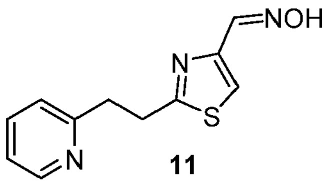

**11**

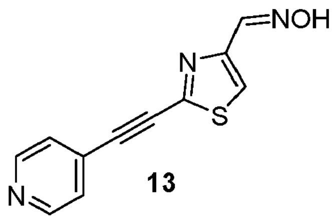

**13**

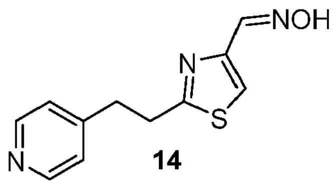

**14**

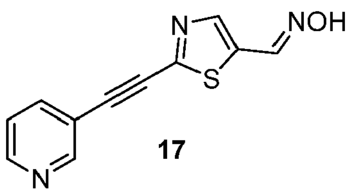

**17**

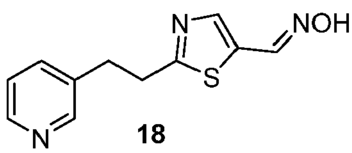

**18**

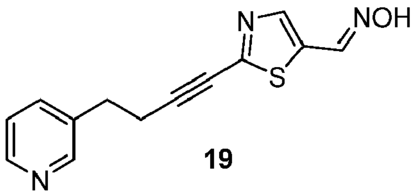

**19**

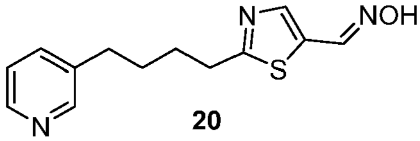

**20**

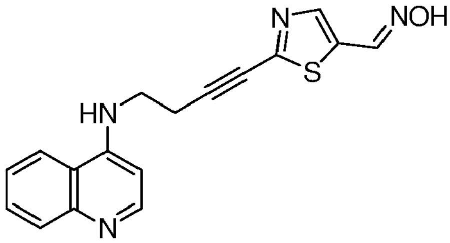

**22**

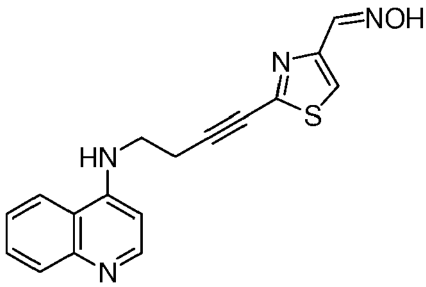

**23**

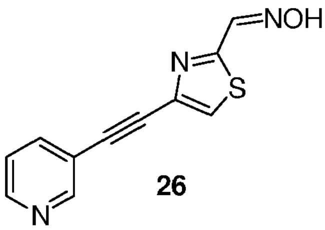

**26**

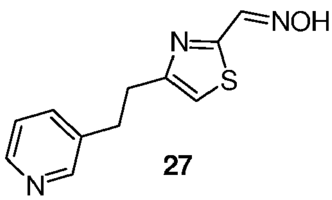

**27**

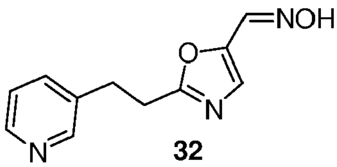

**32**

**9.** Compound according to any one of claims 1 to 8, wherein

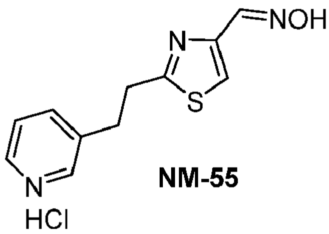

**NM-55**

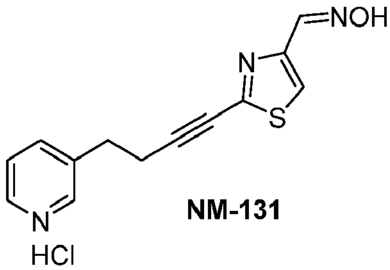

**NM-131**

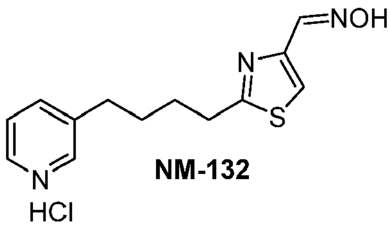

**NM-132**

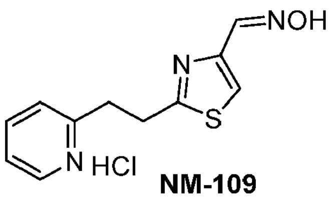

**NM-109**

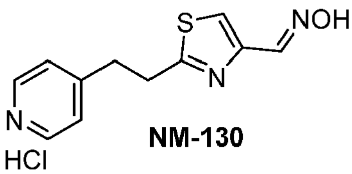

**NM-130**

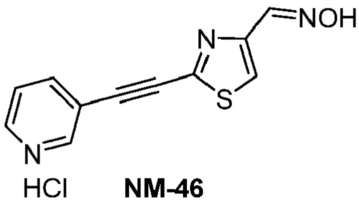

**NM-46**

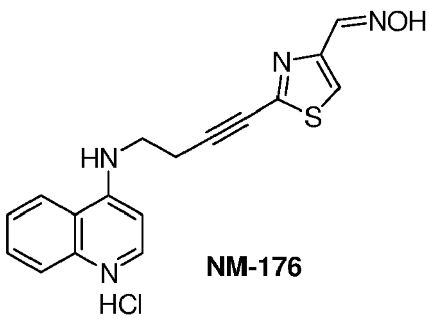

**NM-176**

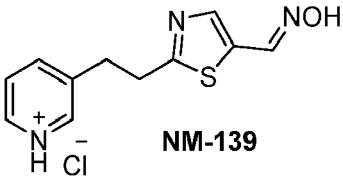

**NM-139**

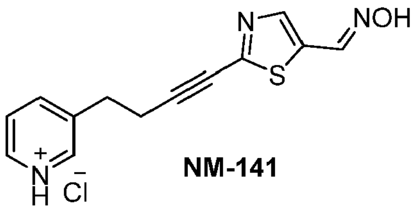

**NM-141**

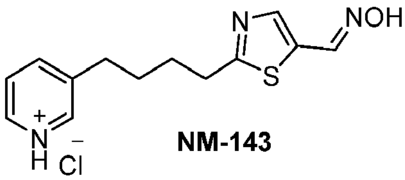

**NM-143**

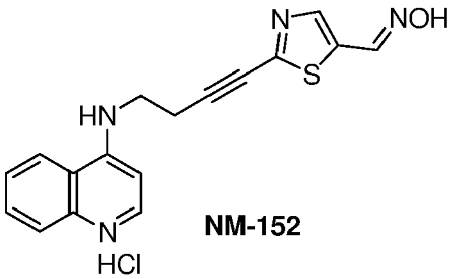

**NM-152**

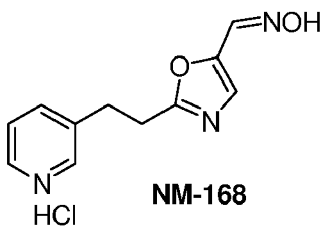

**NM-168**

and

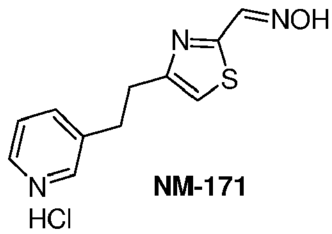

NM-171

**10.** A process for preparing a compound of formula (I) of any one of claims 1 to 9, which comprises the following steps:

- a Sonogashira coupling reaction between a terminal alkyne

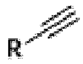

and an isomer of unprotected bromo-oxime-1,3-thiazole or an isomer of unprotected bromo-oxime-1,3-oxazole to obtain the conjugate

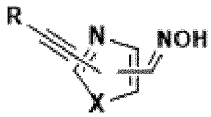

of formula (I);
- optionally, said conjugate is then submitted to hydrogenation to provide the corresponding alkene, and finally the hybrid reactivator

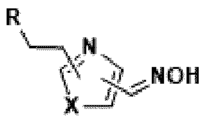

of formula (I).

**11.** A pharmaceutical composition comprising at least one compound of formula (I) according to any one of claims 1 to 9, and at least one pharmaceutically acceptable support.

**12.** The compound according to any one of claims 1 to 9, for use as a medicament.

**13.** The compound according to any one of claims 1 to 9, for use in the treatment of a nervous and/or respiratory failure due to intoxication with at least one organophosphorous nerve agent, by virtue of their reactivation potency of organophosphorous inhibited cholinesterases, including acetylcholinesterase and butyrylcholinesterase.

**14.** The compound according to any one of claims 1 to 9, for use in the treatment of neurological diseases such as Alzheimer’s disease.

**15.** The compound according to any one of claims 1 to 9, for use in the treatment of cancer.

Europäisches Patentamt
European Patent Office
Office européen des brevets

# EUROPEAN SEARCH REPORT

Application Number
EP 20 30 5545

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2015/057822 A1 (UNIV CALIFORNIA [US]; SCRIPPS RESEARCH INST [US]) 23 April 2015 (2015-04-23) * examples 1, 2 * * page 40; compounds RS2-95C, RS2-200D * * claims 2, 3, 10, 12 * ----- | 1-15 | INV. C07D417/08 C07D413/08 C07D417/12 A61K31/427 A61K31/422 A61K31/4439 A61P39/00 A61P39/02 A61P25/28 A61P35/00 |
| X | ZRINKA KOVARIK ET AL: "Reversal of Tabun Toxicity Enabled by a Triazole-Annulated Oxime Library-Reactivators of Acetylcholinesterase", CHEMISTRY - A EUROPEAN JOURNAL, vol. 25, no. 16, 20 November 2018 (2018-11-20), - 18 March 2019 (2019-03-18), pages 4100-4114, XP55734929, DE ISSN: 0947-6539, DOI: 10.1002/chem.201805051 * abstract * * page 4103; table 1; compounds 2A, 1A * * page 4108; table 3; compound 12D * ----- -/-- | 1-15 | |
| | | | TECHNICAL FIELDS SEARCHED (IPC) |
| | | | C07D A61K A61P |

The present search report has been drawn up for all claims

| Place of search | Date of completion of the search | Examiner |
|---|---|---|
| Munich | 29 September 2020 | Cortés Suárez, José |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 30 5545

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/187553 A1 (FMC CORP [US]) 11 October 2018 (2018-10-11)<br>* page 91; table 1; compound with R1=U1(4), R2=2-(OH-N=CH)- *<br>* page 93; table 1; compound with R1=U1(2), R2=4-(OH-N=CH)- *<br>* page 94; table 1; compound with R1=U2(2), R2=4-(OH-N=CH)- *<br>* page 96; table 1; compound with R1=U2(4), R2=2-(OH-N=CH)- *<br>* page 99; table 2; compound with R1=U1(4), R2=2-(OH-N=CH)- *<br>* page 100; table 2; compound with R1=U1(2), R2=4-(OH-N=CH)- *<br>* page 102; table 2; compound with R1=U2(2), R2=4-(OH-N=CH)- *<br>* page 103; table 2; compound with R1=U2(4), R2=2-(OH-N=CH)- *<br>* page 138; index table A; compound 20 *<br>----- | 1,3,5-7 | |
| X | WO 2007/047306 A1 (ELAN PHARM INC [US]) 26 April 2007 (2007-04-26)<br>* page 120; 2nd paragraph; example 14; step 3; compound 4-neopentylthiazole-2-carbaldehyde oxime *<br>----- | 1,5 | TECHNICAL FIELDS SEARCHED (IPC) |

The present search report has been drawn up for all claims

2

| Place of search | Date of completion of the search | Examiner |
|---|---|---|
| Munich | 29 September 2020 | Cortés Suárez, José |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 2 of 2

# ANNEX TO THE EUROPEAN SEARCH REPORT ON EUROPEAN PATENT APPLICATION NO. EP 20 30 5545

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2020

| Patent document cited in search report | Publication date | Patent family member(s) | Publication date |
|---|---|---|---|
| WO 2015057822 A1 | 23-04-2015 | AU 2014337355 A1 | 21-04-2016 |
| | | CA 2926525 A1 | 23-04-2015 |
| | | EP 3057582 A1 | 24-08-2016 |
| | | US 2016256438 A1 | 08-09-2016 |
| | | WO 2015057822 A1 | 23-04-2015 |
| WO 2018187553 A1 | 11-10-2018 | AU 2018249537 A1 | 31-10-2019 |
| | | BR 112019020948 A2 | 05-05-2020 |
| | | CA 3059272 A1 | 11-10-2018 |
| | | CL 2019002853 A1 | 28-02-2020 |
| | | CN 110650956 A | 03-01-2020 |
| | | CO 2019012359 A2 | 17-01-2020 |
| | | EC SP19077553 A | 27-12-2019 |
| | | EP 3606920 A1 | 12-02-2020 |
| | | JP 2020516598 A | 11-06-2020 |
| | | KR 20200003787 A | 10-01-2020 |
| | | PE 20200668 A1 | 11-06-2020 |
| | | PH 12019550206 A1 | 13-07-2020 |
| | | SG 11201909278S A | 28-11-2019 |
| | | TW 201904950 A | 01-02-2019 |
| | | US 2020148672 A1 | 14-05-2020 |
| | | UY 37664 A | 01-10-2019 |
| | | WO 2018187553 A1 | 11-10-2018 |
| WO 2007047306 A1 | 26-04-2007 | CA 2624904 A1 | 26-04-2007 |
| | | EP 1937638 A1 | 02-07-2008 |
| | | JP 2009511589 A | 19-03-2009 |
| | | US 2007149525 A1 | 28-06-2007 |
| | | WO 2007047306 A1 | 26-04-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KATZ et al.** *ChemBioChem.,* 2015, vol. 16, 2205-2215
- **DE KONING et al.** *Eur. J. Med. Chem.,* 2018, vol. 157, 151-160
- **CADIEUX et al.** *Chemico-Biological Interactions,* 2016, vol. 259, 133-141
- **YERRI et al.** *Eur. J. Med .Chem.,* 2018, 4161-4165
- **GALLI et al.** *Chem Med Chem,* 2008, vol. 3, 771-779
- **JOSEPH et al.** *J. Org. Chem.,* 2013, vol. 78, 1670-1676